(19) Europäisches Patentamt / European Patent Office / Office européen des brevets

(11) **EP 3 429 655 B1**

(12) **EUROPEAN PATENT SPECIFICATION**

(45) Date of publication and mention
of the grant of the patent:
**24.02.2021 Bulletin 2021/08**

(21) Application number: **17767077.5**

(22) Date of filing: **02.03.2017**

(51) Int Cl.:
*A61M 1/00* *(2006.01)*    *A61M 27/00* *(2006.01)*
*G16H 20/40* *(2018.01)*    *G16H 40/63* *(2018.01)*

(86) International application number:
**PCT/SG2017/050098**

(87) International publication number:
**WO 2017/160229 (21.09.2017 Gazette 2017/38)**

(54) **A BODY FLUID DRAINAGE DEVICE**

KÖRPERFLÜSSIGKEITSDRAINAGEVORRICHTUNG

DISPOSITIF DE DRAINAGE DE LIQUIDE ORGANIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.03.2016 SG 10201602099Q**

(43) Date of publication of application:
**23.01.2019 Bulletin 2019/04**

(73) Proprietor: **Changi General Hospital Pte Ltd
Singapore 529541 (SG)**

(72) Inventors:
• **QUAH, Lishan Jessica
Singapore 529541 (SG)**

• **CHING, Tsz Him
Singapore 529541 (SG)**

(74) Representative: **Loyer & Abello
9, rue Anatole de la Forge
75017 Paris (FR)**

(56) References cited:
WO-A1-2011/037838     WO-A1-2011/037838
WO-A1-2011/107972     WO-A1-2011/118888
WO-A1-2015/197463     WO-A2-01/21129
US-A1- 2014 066 903     US-A1- 2014 194 840
US-A1- 2014 194 840     US-B2- 9 186 449

**Description**

**TECHNICAL FIELD**

The present invention relates broadly to a body fluid drainage device for draining a fluid from a body cavity.

**BACKGROUND**

Body cavities accumulate fluid in diseased states of a mammalian body. Fluid accumulation may occur, for example, in the thoracic cavity or abdomen of a mammalian body. Common causes of fluid accumulation include heart failure, kidney failure, liver failure or cancers etc.

A pleural cavity is a space between a parietal and a visceral pleural within a human thorax. The space is disposed between the lungs and the chest wall. The pleural cavity typically contains a small amount of fluid for, for example, lubrication for chest expansion during normal respiration. Due to diseased states of a body, an abnormal amount of fluid e.g. air and/or liquid may accumulate in the pleural cavity. Air in the pleural cavity is known as pneumothorax. Abnormal fluid collection in the pleural cavity is known as pleural effusion (PE).

For another disorder, accumulation of fluid within a peritoneal cavity typically results in ascites. Occurrence of ascites is typically due to portal hypertension resulting from cirrhosis. Other common causes of ascites include malignancy and heart failure etc.

A current practice of fluid drainage from a body cavity uses a catheter that is inserted into the body cavity. The catheter is then connected to a drainage box. A medical caregiver or practitioner is typically required to begin the fluid drainage. Constant monitoring by a caregiver such as a physician or a nurse is typically required throughout the entire process of fluid drainage. Once a desired volume of fluid drainage is observed by the caregiver, the catheter is manually clamped using a mechanical tool by the caregiver to stop drainage. As such, the current fluid drainage process is heavily dependent on human intervention and the efficiency of the current fluid drainage process may vary between caregivers. There is therefore a high degree of subjectivity and a potential occurrence of human error.

Currently, constant monitoring of fluid drainage is typically needed because the pressure in a body cavity is typically unknown. In practice, when the pressure in the body cavity is substantially lower than atmospheric pressure, accumulated fluid in the body cavity is prevented from draining. On the other hand, when the pressure in the body cavity is substantially higher than atmospheric pressure, accumulated fluid may flow in significant volumes out of the body cavity.

Mismanagement of fluid drainage from the body cavity may pose a number of dangers to a mammalian body. One such danger is the risk of having a condition known as re-expansion pulmonary edema (RPE) when draining fluid from the pleural cavity. This condition occurs when there is rapid expansion of a collapsed lung and may lead to fatal consequences.

Another danger arising from mismanaged fluid drainage may occur during removal of ascitic fluid. For example, during abdominal paracentesis to remove ascitic fluid, low blood pressures may occur when large volumes of fluid are removed without adequate management.

In view of the above, it has been recognized by the inventors that current practices of fluid drainage from a body cavity may be inconsistent and may typically be highly dependent on the monitoring and skills of different caregivers. In addition, during fluid drainage, a patient is also typically confined to a bed/couch in a medical facility as the current drainage equipment are non-portable and because monitoring is required by a caregiver.

Therefore, there exists a need for a body fluid drainage device for draining a fluid from a body cavity that seek to address one or more of the problems above.

US 2014/194840 A1 discloses a portable body fluid collection device comprising an apparatus for collection of body fluids that includes a drainage tube, a fluid collection chamber in fluid communication with the drainage tube, and at least one strap wherein the strap can be worn by the patient to support the weight of the body fluid drain control apparatus.

WO 2011/037838 A1 discloses a device for regulating cerebral spinal fluid drainage amounts from brain, spine, tissue or organs of a patient, wherein the device is a portable external gravity-based device.

US 9186449 B2 discloses a dialysis machine support assembly comprising a platform configured to support a dialysis machine, a drive assembly configured to move the platform vertically, and a control unit configured to monitor input data from a sensor of the dialysis machine when the dialysis machine is supported by the platform. The control unit can operate the drive assembly to adjust a vertical position of the platform based on the input data.

WO 2015/197463 A1 discloses a device for suctioning and passing blood during a surgical operation and to a secretion collection container.

## SUMMARY

The invention is defined by the appended claims. In accordance with an aspect, there is provided a body fluid drainage device for draining a fluid from a body cavity, the device comprising, a channel switching module for coupling to a fluid containment device, the channel switching module for controlling a direction of flow of the fluid with respect to the fluid containment device; a flow rate actuator for coupling to the fluid containment device, the flow rate actuator for controlling a rate of fluid flow into the fluid containment device; a processing module coupled to the channel switching module, the processing module being configured to control a fluid flow between the body cavity and the fluid containment device and to control a fluid flow from the fluid containment device using the channel switching module; wherein the processing module is capable of controlling the fluid flow between the body cavity and the fluid containment device based on a pre-determined fluid volume in the fluid containment device; and wherein the fluid containment device comprises a syringe.

The body fluid drainage device may further comprise the fluid containment device, the fluid containment device being removably coupled to the flow rate actuator.

The fluid containment device may be removably coupled to the channel switching module.

The channel switching module, the flow rate actuator or both may be adapted to be removably coupled to the processing module.

The processing module may be configured to determine whether a fluid volume in the fluid containment device matches the pre-determined fluid volume, and if the fluid volume in the fluid containment device matches the pre-determined fluid volume, the processing module is configured to stop fluid flow between the body cavity and the fluid containment device.

The processing module may be configured to stop fluid flow between the body cavity and the fluid containment device using the flow rate actuator, or using channel switching module or both.

The determination of whether a fluid volume in the fluid containment device matches the pre-determined fluid volume may be based on a maximum volume of the fluid containment device.

The determination of whether a fluid volume in the fluid containment device matches the pre-determined fluid volume may be based on the rate of fluid flow controlled by the flow rate actuator.

The rate of fluid flow controlled by the flow rate actuator may be correlated to an advancement distance of the fluid flow in the fluid containment device.

The channel switching module may be capable of switching between at least two channels; further wherein in a first operation state, the processing module is configured to instruct the channel switching module to switch to a first channel to allow fluid communication from the body cavity to the fluid containment device through the first channel; and in a second operation state, the processing module is configured to instruct the channel switching module to switch to a second channel to allow fluid communication from the fluid containment device through the second channel.

In the second operation state, the processing module may be configured to actuate expelling of fluid from the fluid containment device with an actuation of the flow rate actuator in reverse motion.

The processing module may be configured to determine whether a pre-determined total volume of fluid drainage has been achieved, said determination based on determining a number of times the pre-determined fluid volume in the fluid containment device is obtained.

The said determination may be further based on a number of switches of the channel switching module to allow fluid flow from the fluid containment device.

The body fluid drainage device may further comprise an input module, the input module being configured to allow input to the processing module of the pre-determined total volume of fluid drainage and input to the processing module of the rate of fluid flow into the fluid containment device.

The body fluid drainage device may further comprise a storage component, the storage component being coupled to the channel switching module for receiving fluid flow from the fluid containment device.

The body fluid drainage device may further comprise a pressure sensor coupled to the channel switching module for measuring a pressure in the body cavity.

The body fluid drainage device may further comprise one or more force sensors coupled to the fluid containment device for measuring a pressure in the body cavity.

The processing module may be further configured to control the fluid flow between the body cavity and the fluid containment device based on the measured pressure.

The flow rate actuator may comprise a force transmittal member coupled to the fluid containment device and coupled to the processing module; wherein the processing module is configured to control the fluid flow into the fluid containment device and expelled from the fluid containment device via a displacement of the force transmittal member.

## BRIEF DESCRIPTION OF THE DRAWINGS

Exemplary embodiments of the invention will be better understood and readily apparent to one of ordinary skill in the art from the following written description, by way of example only, and in conjunction with the drawings, in which:

FIG. 1 is a schematic block drawing of a body fluid drainage device for draining a fluid from a body cavity in an exemplary embodiment.

FIG. 2 is a perspective exploded view of a body fluid drainage device in an exemplary embodiment.

FIGS. 3A to 3D are schematic drawings for illustrating an operation of a body fluid drainage device in an exemplary embodiment.

FIG. 4A is a perspective view of a channel switching module of a body fluid drainage device in an exemplary embodiment.

FIG. 4B is an exploded view of the channel switching module of FIG. 4A.

FIG. 5C is a perspective view of the fluid drainage module of FIG. 5B with a plunger member in an extended state.

FIG. 5D is a top view of the fluid drainage module of FIG. 5C with the plunger member in a fully extended state.

FIG. 5E is an exploded view of the fluid drainage module of FIG. 5A to 5D.

FIG. 5F is a schematic drawing illustrating positions of two contact switches in a fluid drainage module in an exemplary embodiment.

FIG. 5G is a schematic drawing illustrating positions of the two contact switches in the fluid drainage module corresponding to a covering member being in a fully extended state.

FIG. 6A is a perspective view of a docking module of a body fluid drainage device in an exemplary embodiment.

FIG. 6B is an exploded view of the docking module of FIG. 6A.

FIG. 7A is a schematic drawing illustrating a body fluid drainage device in use in an exemplary embodiment.

FIG. 7B is a schematic drawing of the body fluid drainage device.

FIG. 8 is a top view of a body fluid drainage system for draining a fluid from a body cavity in an exemplary embodiment.

FIG. 9 is a schematic diagram for illustrating a gear train assembly.

FIG. 10 is a schematic flowchart for illustrating an operation of a body fluid drainage device in an exemplary embodiment.

FIG. 11 is a schematic flowchart for illustrating a method of forming a body fluid drainage device for draining a fluid from a body cavity in an exemplary embodiment.

## DETAILED DESCRIPTION

Exemplary embodiments described herein may provide a body fluid drainage device for draining a fluid from a body cavity.
The term "body cavity" as used in this description refers to a body cavity or part of an animal body.
The term "coupling" of different modules or components as used in this description may refer to removable coupling or integral coupling.
The term "fluid" as used in this description may comprise a liquid, a gas or a mixture of both liquid and gas.
The term "coupling" of different modules or components as used in this description may refer to removable coupling or integral coupling.

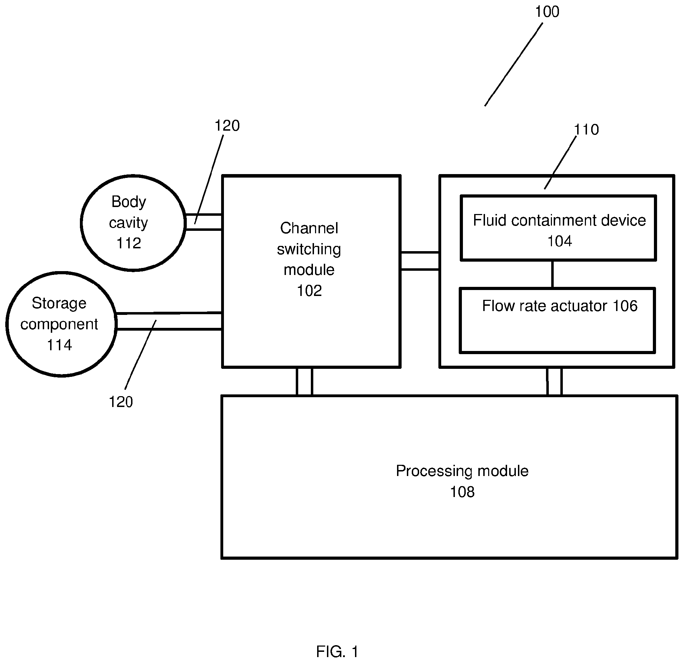

FIG. 1 is a schematic block drawing of a body fluid drainage device for draining a fluid from a body cavity in an exemplary embodiment. In the exemplary embodiment, the body fluid drainage device 100 comprises a channel switching module 102, a flow rate actuator 106 and a processing module 108. A fluid containment device 104 may be separately provided to removably couple to the channel switching module 102 and to removably couple to the flow rate actuator 106.

The channel switching module 102 controls a direction of flow of the fluid 120 with respect to the fluid containment device 104. The flow rate actuator 106 controls or calibrates a rate of fluid flow into the fluid containment device 104.

In the exemplary embodiment, the fluid containment device 104 and the flow rate actuator 106 form a fluid drainage module 110. The processing module 108 is coupled to the channel switching module 102 and the fluid drainage module 110. For the body fluid drainage device 100, at least the fluid drainage module 110 is configured to be removably coupled to the processing module 108.

The channel switching module 102 is adapted to be removably coupled to a body cavity 112, e.g. via a catheter providing fluid communication from the body cavity 112. The channel switching module 102 is also removably coupled to a storage component 114.

The processing module 108 is configured to control a fluid flow between the body cavity 112 and the fluid containment device 104. The processing module 108 is also configured to control a fluid flow from the fluid containment device 104. The processing module 108 is also configured to control the operation of the flow rate actuator 106. Thus, the processing module 108 is capable of instructing/using the channel switching module 102 to allow fluid flow between the body cavity 112 and the fluid containment device 104. The processing module 108 is also capable of instructing/using the channel switching module 102 to allow fluid flow from the fluid containment device 104, e.g. to expel fluid from the fluid containment device 104. The processing module 108 is capable of controlling the fluid flow between the body cavity 112 and the fluid containment device 104 based on or depending on whether a pre-determined or known fluid volume has been obtained in the fluid containment device 104.

In the exemplary embodiment, in use, fluid communication of the fluid 120 is controlled to the fluid containment device 104 from the body cavity 112 by an operation state of the channel switching module 102. Fluid communication of the fluid 120 is also controlled from or out of the fluid containment device 104 to the storage component 114 by another operation state of the channel switching module 102. The channel switching module 102 switches fluid communication of the fluid containment device 104 between the body cavity 112 and the storage component 114.

In the exemplary embodiment, the channel switching module 102, on instruction by the processing module 108, controls the switching of the fluid communication based on whether a desired or pre-determined or known fluid volume has been obtained in the fluid containment device 104. In the exemplary embodiment, the fluid 120 is allowed to be drained from the body cavity 112 into the fluid containment device 104 with a desired flow rate monitored or implemented by the flow rate actuator 106 on instruction by the processing module 108. Once a pre-determined fluid volume is determined to be obtained in the fluid containment device 104 on instruction by the processing module 108, the channel switching module 102, on instruction by the processing module 108, switches to allow the fluid 120 to be drained or expelled from the fluid containment device 104, for example, to the storage component 114. The channel switching module 102, on instruction by the processing module 108, controls the switching until a desired total volume of the fluid 120 is drained into the storage component 114 from the body cavity 112. The total volume may be calculated from a number of times the pre-determined fluid volume is obtained in the fluid containment device 104.

In some exemplary embodiments, the number of times the pre-determined fluid volume is obtained in the fluid containment device 104 corresponds to the number of switches of the channel switching module 102 to drain the fluid 120 into the storage component 114.

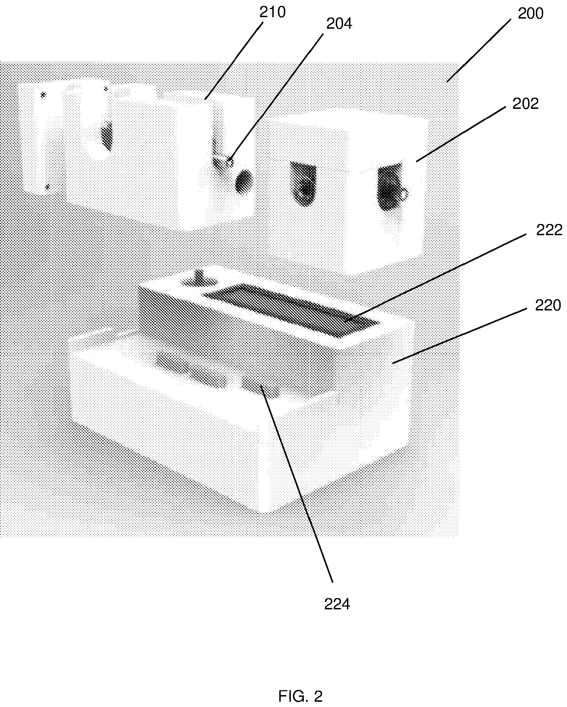

FIG. 2 is a perspective exploded view of a body fluid drainage device in another exemplary embodiment. In the exemplary embodiment, the body fluid drainage device 200 functions substantially identically to the body fluid drainage device 100 described with reference to FIG. 1.

The body fluid drainage device 200 comprises a channel switching module 202 that is able to be removably coupled to a fluid drainage module 210. The body fluid drainage device 200 further comprises a docking module 220. The docking module 220 comprises a processing module (not shown), a display component 222 and connectors e.g. 224. The channel switching module 202 and the fluid drainage module 210 are able to be removably coupled to the docking module 220 via the connectors 224.

The fluid containment device 204 is shown coupled thereto within the fluid drainage module 210 but it will be appreciated that the fluid containment device 204 may be provided separately from the body fluid drainage device 200 and may not form part of the body fluid drainage device 200. It will also be appreciated that a storage component to contain expelled fluid from the fluid containment device 204 and that is suitable to be coupled to the channel switching module 202 may be provided separately from the body fluid drainage device 200 and may not form part of the body fluid drainage device 200.

In the exemplary embodiment, the fluid containment device 204 is in the form of a syringe, but it will be appreciated that the fluid containment device 204 is not limited as such. For example, the fluid containment device 204 may be a bag or container calibrated for measuring a volume of a fluid collected in the fluid containment device 204. The display component 222 may be, but is not limited to, a liquid crystal display (LCD) to visually display parameters or settings inputted by a user and/or the current operating status of the body fluid drainage device 200.

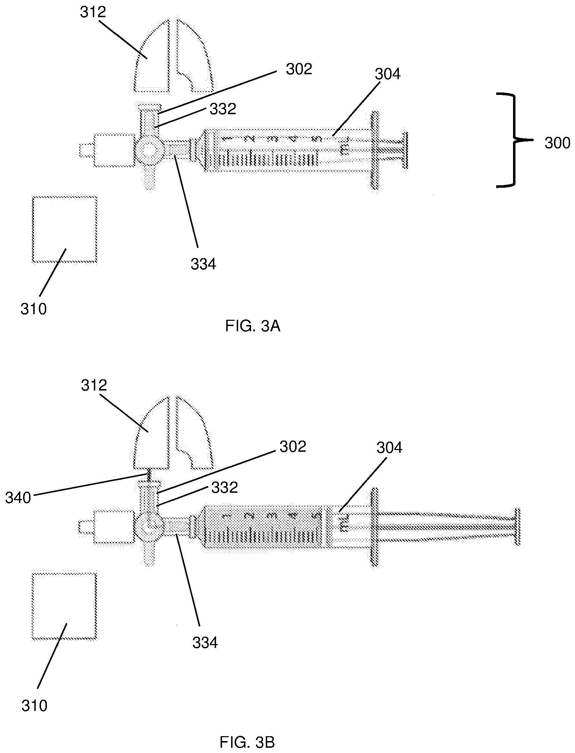

FIGS. 3A to 3D are schematic drawings for illustrating an operation of a body fluid drainage device in an exemplary embodiment. In the exemplary embodiment, a body cavity 312 in the form of a pleural cavity is shown. A storage component 310 in the form of a drainage box/bag is also shown. For ease of understanding, only a channel switching module 302 of the body fluid drainage device 300 and a fluid containment device 304 coupled thereto the body fluid drainage device 300 are shown.

In the exemplary embodiment, the channel switching module 302 comprises at least two channels 332, 334. The channel switching module 302 may be in the form of, but not limited to, a three-way tap/valve. The sterile parts may be the fluid containment device (e.g. a syringe 304) and the at least two channels 332, 334 of the channel switching module 302 (e.g. a three-way valve). The channel switching module 302 is coupled to the body cavity 312 using a catheter (not shown) to allow fluid communication from the body cavity 312 to the fluid containment device 304. The volume of the fluid containment device 304 may range from about 5ml to about 50ml and therefore, the body fluid drainage device 300 for housing or coupling to the fluid containment device 304 is portable and pocket-sized.

During use of the body fluid drainage device 300, a user inputs a desired maximum/total volume of fluid drainage and a desired flow rate (e.g. in milliliters per second) to a processing module (not shown). After the desired maximum/total volume of fluid drainage and the desired flow rate are inputted into the processing module, no human intervention is needed for monitoring of the body fluid drainage device or the aforementioned desired parameters.

In FIG. 3A, the processing module is capable of determining whether a pre-determined volume of fluid in the fluid containment device 304 has been obtained. For example, the pre-determined volume may be the maximum volume of the fluid containment device 304. As another example, the determination of whether the pre-determined volume is obtained may be from a calculation using the rate of fluid flow and the time elapsed of operation of the body fluid drainage device 300. In another example, the determination of whether the pre-determined volume is obtained may be correlated to an advancement distance of the fluid flow in the fluid containment device. For example, the fluid volume may be a correlation of the linear distance moved by the plunger of a syringe.

In FIG. 3B, the channel switching module 302 is shown to be in a first operation state during operation of the body fluid drainage device. In the first operation state, the processing module uses the channel switching module 302 to control fluid flow between the body cavity 312 and the fluid containment device 304. Thus, body fluid 340 is allowed to flow from the body cavity 312 through a first channel 332 into the fluid containment device 304. The processing module controls the flow rate of the fluid into the fluid containment device 304 using the flow rate actuator (not shown) and based on the inputted pre-determined flow rate. For example, the processing module may control, via voltage control, a motor coupled to the flow rate actuator to allow or control a rate of fluid flow into the fluid containment device 304.

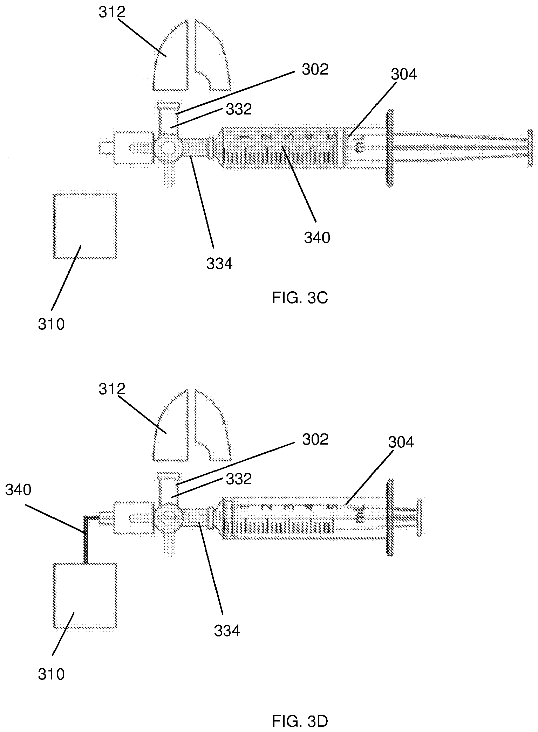

Next, when a fluid volume in the fluid containment device 304 is matched to a pre-determined volume by the processing module, for example the fluid containment device 304 is filled fully with the fluid, the channel switching module 302 switches from the first operation state to a second operation state, as shown in FIG. 3C. That is, a pre-determined volume of fluid in the fluid containment device 304 has been obtained.

It will be appreciated that the transition to the second operation state comprises the processing module instructing the fluid drainage module (not shown) comprising the flow rate actuator (not shown) to stop further drainage of fluid from the body cavity. In addition or as an alternative, further drainage of fluid from the body cavity may be stopped by the processing module instructing the channel switching module to switch from the first operation state to the second operation state.

The processing module is also configured to count or record the number of instances of the pre-determined volume of fluid in the fluid containment device 304 being obtained. In some exemplary embodiments, the processing module is also configured to count or record the number of switches from the first operation state to the second operation state.

In the second operation state, the processing module uses the channel switching module 302 to control fluid flow from the fluid containment device 304. Thus, body fluid 340 is allowed to flow out of, or be expelled from, the fluid containment device 304 through a second channel 334.

In FIG. 3D, the channel switching module 302 is shown to be in the second operation state during operation of the body fluid drainage device. Body fluid 340 is shown to flow from the fluid containment device 304 through the second channel 334 of the channel switching module 302, for example, into the storage component 310. In the exemplary embodiment, the storage component 310 is shown coupled to the channel switching module 302.

In the exemplary embodiment, the channel switching module 302 is capable of switching interchangeably between the first operation state and the second operation state. The processing module is configured to instruct the switching between the operation states to control a fluid flow between the body cavity 312 and the fluid containment device 304 based on whether the pre-determined fluid volume in the fluid containment device 304 is obtained. The processing module is also configured to stop fluid communication from the body cavity 312 through the first channel 332 when the processing module detects that a pre-determined total volume of fluid drainage is obtained. The total volume of fluid drainage may be derived by a number of times the pre-determined fluid volume is obtained in the fluid containment device 304. In some exemplary embodiments, the number of times the pre-determined fluid volume is obtained in the fluid containment device 304 corresponds to the number of switches of the channel switching module 302 to allow the pre-determined fluid volume in the fluid containment device 304 to flow or expel from or out of the fluid containment device 304.

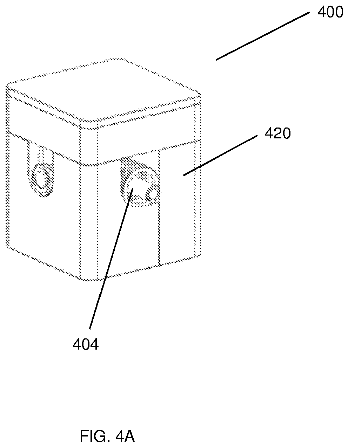

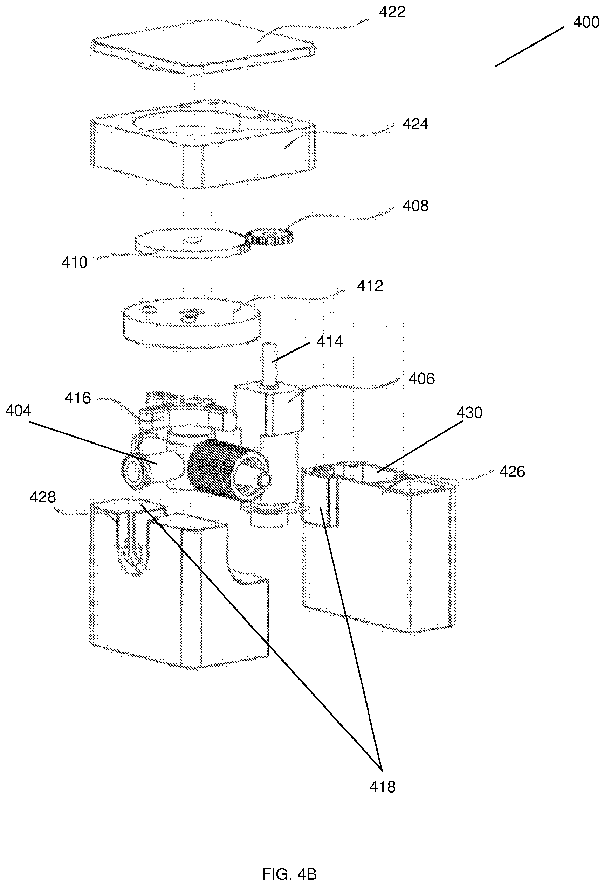

FIG. 4A is a perspective view of a channel switching module 400 of a body fluid drainage device in an exemplary embodiment. FIG. 4B is an exploded view of the channel switching module 400. In the exemplary embodiment, the channel switching module 400 functions substantially identically to the channel switching module 202 described with reference to Fig. 2. The channel switching module 400 comprises a valve 404, a first motor assembly 406, a first gear 408, a second gear 410, a rotable disc 412 and a first housing 420. The valve 404 may be, but is not limited to, a three-way tap.

The first housing 420 comprises a first intermediate portion 424, a first bottom portion 426 and a second bottom portion 428. The first housing 420 may further comprise a first top portion 422. The first bottom portion 426 is provided with an opening 430 to receive the first motor assembly 406. The second bottom portion 428 is adapted to receive and accommodate the valve 404 within the first housing 420.

The first motor assembly 406 comprises a gearbox (not shown) and an encoder (not shown). The first motor assembly 406 also comprises a drive shaft 414.

The first gear 408 and the second gear 410 are in rotational contact with each other to form a gear train. The first gear 408 is coupled to the drive shaft 414 of the first motor assembly 406. The second gear 410 is fixedly coupled to the rotable disc 412. The rotable disc 412 is coupled to a trigger member 416 of the valve 404.

During use, energy from the first motor assembly 406 is transferred through the drive shaft 414 to the first gear 408 which in turn causes the second gear 410 to rotate. The rotable disc 412 rotates together with the second gear 410 and causes the trigger member 416 to turn and switch the channel switching module 400 interchangeably between a first operation state and a second operation state.

The first bottom portion 426 is coupled to the first intermediate portion 424 by, for example, connecting pins. The first top portion 422 may be coupled to the first intermediate portion 424 by connecting pins (not shown). The first bottom portion 426 and the second bottom portion 428 are removably coupled to each other, for example, by a magnet assembly 418. The first bottom portion 426 and the second bottom portion 428 are therefore easily separated from each other for a user/caregiver to access or, for example, to replace the valve 404.

In the exemplary embodiment, the gear module value of the first gear 408 is 0.5 and the number of teeth of the first gear 408 is 18 ($N_1$). The gear module value of the second gear 410 is 0.5 and the number of teeth of the second gear 410 is 48 (N2). The gear ratio of the first motor assembly 406 is 297.92:1.

For every quarter turn of the valve 404, the first gear 408 turns:

$$0.25 \times \frac{48}{18} = \frac{2}{3} turns$$

In association, the motor in the first motor assembly 406 turns:

$$297.92 \times \frac{2}{3} = 198.61 revolutions$$

In association, the encoder in the first motor assembly 406 records:

$$198.61 \times 3 = 595.84 \ counts$$

Therefore, it may be provided for a processing module to instruct the first motor assembly 406 to be able to switch and turn the valve 404.

In the exemplary embodiment, the length, breadth and height of an exterior of the first housing 420 of the channel switching module 400 are about 35mm, 35mm and 42mm respectively. Therefore, the channel switching module 400 is suitably portable and smaller than a typical palm size and/or pocket-size.

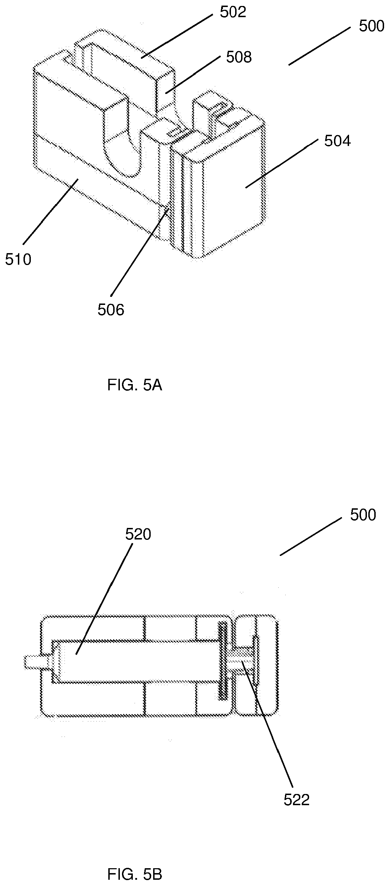

FIG. 5A is a perspective view of a fluid drainage module of a body fluid drainage device in an exemplary embodiment. The fluid drainage module 500 is shown without a fluid containment device being coupled thereto. FIG. 5B is a top view of the fluid drainage module in the exemplary embodiment with a fluid containment device coupled thereto. In the exemplary embodiment, the fluid drainage module 500 functions substantially identically to the fluid drainage module 210 described with reference to Fig. 2. The fluid containment device 520 may be provided separately from the fluid drainage module 500 and may not form part of the resultant body fluid drainage device (compare body fluid drainage device 200 of FIG.2).

In the exemplary embodiment, the fluid containment device 520 is in the form of a syringe.

The fluid drainage module 500 comprises a second housing 502, a covering member 504 and a force transmittal member 506. The force transmittal member 506 functions as a flow rate actuator. The second housing 502 is adapted to removably receive the fluid containment device 520. The second housing 502 is coupled to the covering member 504 by the force transmittal member 506. The force transmittal member 506 may be, but is not limited to, an aluminum tube. The covering member 504 is configured to receive a plunger member 522 of the syringe. In FIG. 5B, the plunger member 522 is in a non-extended or unextended state, for example, when there is no fluid in the syringe.

The use of the force transmittal member 506 to actuate movement of the covering member 504 controls a rate of flow of fluid into the syringe.

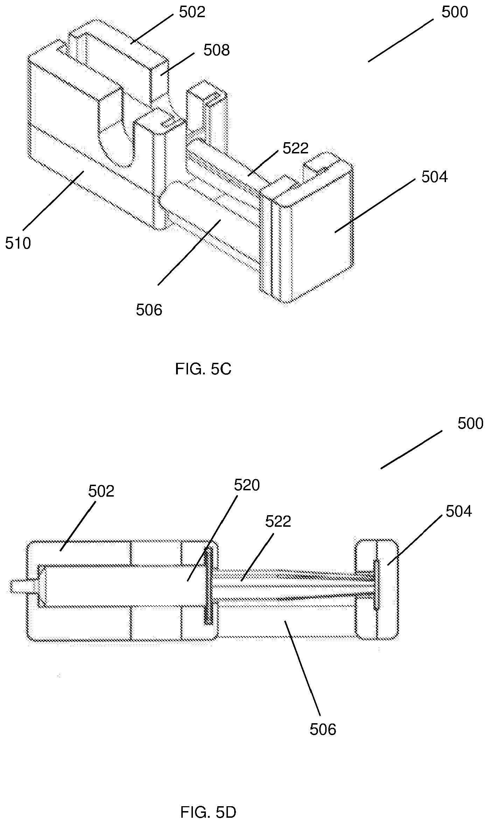

FIG. 5C is a perspective view of the fluid drainage module 500 with the plunger member 522 in an extended state. For example, the plunger member 522 is in an extended state when there is fluid contained in the syringe. FIG. 5D is a top view of the fluid drainage module 500 with the plunger member 522 is in a fully extended state. For example, the plunger member 522 is in a fully extended state when fluid contained in the syringe fills the maximum volume of the syringe.

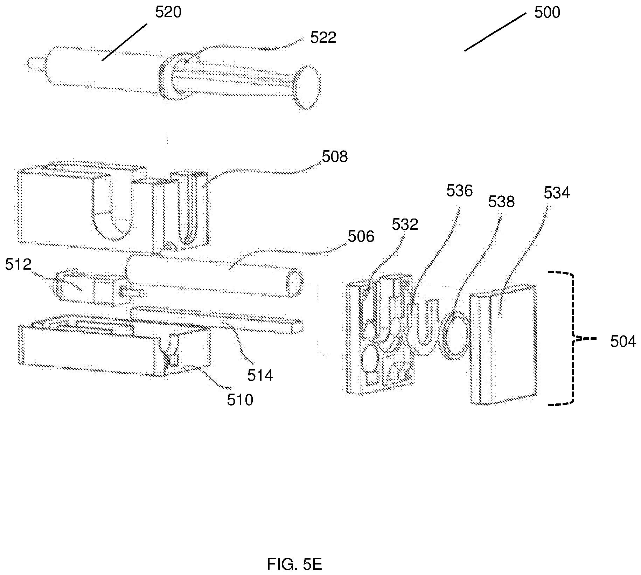

FIG. 5E is an exploded view of the fluid drainage module 500, as described with reference to FIG. 5A to 5D. The second housing 502 of the fluid drainage module 500 comprises a second top portion 508 and a third bottom portion 510. The second top portion 508 and the third bottom portion 510 may be coupled to each other after assembly of the components of the fluid drainage module 500. The covering member 504 comprises a first panel 532, a second panel 534, a first force distribution member 536 and a second force distribution member 538. The first force distribution member 536 and the second force distribution member 538 are used to distribute force onto force sensors (not shown) in an even manner.

The fluid drainage module 500 further comprises a second motor assembly 512 and a gear rack 514. The gear rack 514 is fixedly coupled to the force transmittal member 506.

During use, the force transmittal member 506 transmits a force exerted from a movement of the gears of the second motor assembly 512 to the plunger member 522. Extended states of the plunger member 522 are as shown in FIGS. 5C and 5D. In more detail, rotational force of the gears of the second motor assembly 512 is translated via the gear rack 514 to cause displacement of the force transmittal member 506. Due to the movement of the force transmittal member 506, the covering member 504 displaces in the same direction as the displacement of the force transmittal member 506. When the covering member 504 moves, the plunger member 522 displaces in the same direction as the covering member 504. As a consequence, fluid flows into the fluid containment device 520 with an advancement distance corresponding to the displacement distance of the plunger member 522. In this way, the fluid volume may be a correlation of the linear distance moved by the plunger of a syringe as controlled or calibrated by the force transmittal member 506 functioning as the flow rate actuator.

The fluid containment device 520 is capable of discharging fluid when it is determined by a processing module that the fluid in the fluid containment device 520 reaches or matches a pre-determined volume. For example, the pre-determined volume may be, but is not limited to, a maximum volume of the fluid containment device 520. The total volume of fluid drained from the body cavity (i.e. the pre-determined total volume) is achieved based on the number of times the fluid containment device 520 is determined to contain a pre-determined volume of the fluid. For example, if the pre-determined total volume is to be 1.5 liters, the fluid containment device 520 with a volume of a 5ml can discharge the fluid 300 times, for example, into a collection box, with each discharge based on a maximum volume of the fluid containment device 520 being achieved (as the pre-determined volume).

In the exemplary embodiment, the flow rate actuator controls a flow of fluid into the fluid containment device 520. When the pressure in the body cavity is at a significantly higher level than atmospheric pressure, the body fluid typically flows out from the body cavity at an unknown rate which may not be medically acceptable. In this scenario, the flow rate actuator functions to regulate the flow rate of the fluid from the body cavity into the fluid containment device 520.

In the description below, it will be described exemplarily how the processing module may determine that a pre-determined volume of fluid is within the fluid containment device 520. The determination may be by, for example but not limited to, a sensor, such as a contact sensor or switch, or by a linear distance moved by e.g. a plunger of a syringe.

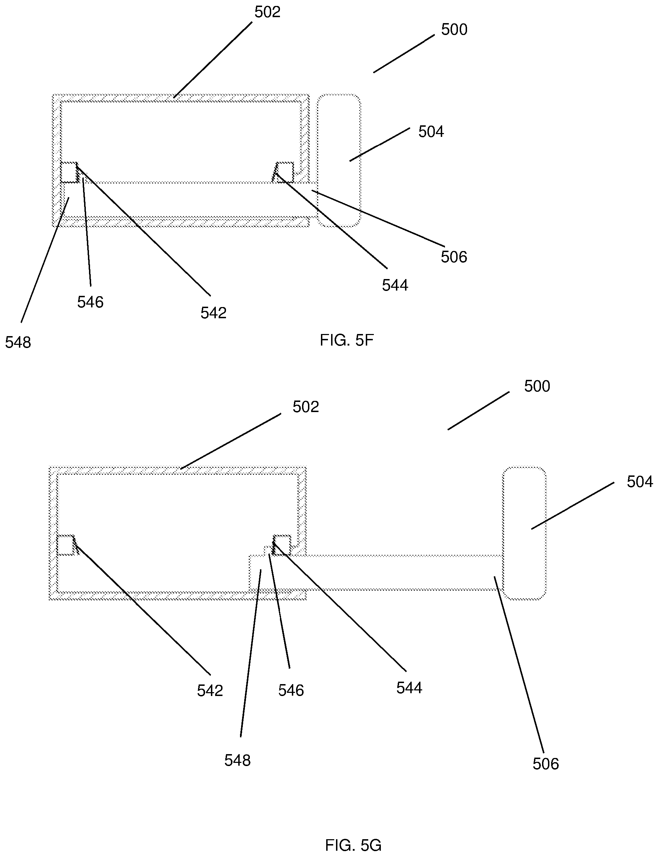

FIG. 5F is a schematic drawing illustrating positions of two contact switches in a fluid drainage module in an exemplary embodiment. The covering member 504 is shown being in a non-extended/unextended state. FIG. 5G is a schematic drawing illustrating positions of the two contact switches in the fluid drainage module corresponding to the covering member 504 being in a fully extended state. For the ease of illustration, the fluid containment device is not shown in FIGS. 5F and 5G.

As shown in FIGS. 5F and 5G, a first contact switch 542 and a second contact switch 544 are used in the fluid drainage module 500 to inform a processing module that the body fluid in the fluid containment device (e.g. 520 in FIGS. 5B, 5D and 5E) reaches a pre-determined volume in the fluid containment device (e.g. 520 in FIGS. 5B, 5D and 5E). The pre-determined volume may be a maximum volume of the fluid containment device (e.g. 520 in FIGS. 5B, 5D and 5E). The force transmittal member 506 comprises a protruding part 546 at a first end 548. The protruding part 546 is adapted to mechanically contact the first contact switch 542 to cause or bias the first contact switch 542 into a closed position, for example when the fluid containment device (e.g. 520 in FIGS. 5B, 5D and 5E) is empty or is in a non-extended or unextended state.

When the fluid containment device (e.g. 520 in FIGS. 5B, 5D and 5E) is in a non-extended or unextended state, the force transmittal member and the covering member 504 are at an initial reference position. The protruding part 546 comes into mechanical contact with the first contact switch 542 to cause or bias the first contact switch 542 into a close position. The second contact switch 544 is in an open position. The force transmittal member 506 functioning as the flow rate actuator may be instructed by the processing module to control or calibrate the flow of body fluid into the fluid containment device.

When body fluid flows into the fluid containment device, the force transmittal member 506 displaces and causes the covering member 504 to displace in the same direction. The plunger member (e.g. 522 in FIGS. 5B to 5E) is extended. The protruding part 546 releases contact with the first contact switch 542 and the first contact switch 542 changes to an open position. When the plunger member (e.g. 522 in FIGS. 5B to 5E) reaches its fully extended state, the protruding part 546 is in mechanical contact with the second contact switch 544 and causes the second contact switch 544 into a closed position. This causes the processing module to stop further drainage of fluid from the body cavity and to instruct a channel switching module to switch and allow fluid flow from the fluid containment device 520 instead of allowing fluid flow between the body cavity and the fluid containment device 520.

In the exemplary embodiment, the processing module also transmits information to the second motor assembly 512 to cause the force transmittal member 506 to move or displace the plunger member 522 towards its initial reference position (i.e. non-extended state). The movement of the plunger member 522 causes fluid to be expelled from the fluid containment device 520. When the plunger member 522 reaches its initial reference position (i.e. non-extended state), the first contact switch 542 closes to inform the processing module that the pre-determined volume of fluid has been expelled. The processing module may instruct the channel switching module to switch to allow fluid flow between the body cavity and the fluid containment device 520 to allow a next cycle of fluid drainage. Alternatively, the processing module may also instruct the fluid drainage module to stop drainage if it is determined that a pre-determined total volume of fluid has been drained.

The contact switches 542, 544 may also act as a safety feature to stop the body fluid drainage device from continuing to drain body fluid from the body cavity. For example, when the plunger is over-extended from the syringe, the second contact switch 544 closes and prevents the body fluid from continuing to be drained from the body cavity.

In another exemplary embodiment, when the number of turns made by a motor in a second motor assembly and the linear distance moved by a syringe are detected to have reached a predetermined value, and the pre-determined value/distance corresponds or correlates to a volume, the syringe is switched to discharge the body fluid. In this example, the syringe does not have to reach its maximum volume before the body fluid is discharged out of the syringe. That is, the determination of whether a fluid volume in the fluid containment device matches the pre-determined fluid volume is based on the rate of fluid flow controlled by the flow rate actuator. The determination is also correlated to an advancement distance of the fluid flow in the fluid containment device, as allowed by the displacement or linear distance moved by a plunger of a syringe.

In the exemplary embodiment, the length, breadth and height of an exterior of the second housing 502 of the fluid drainage module 500 are about 68mm, 35mm and 42mm respectively. The length, breadth and height of an exterior of the covering member 504 are about 15mm, 35mm and 42mm respectively.

Therefore, the fluid drainage module 500 is suitably portable and smaller than a typical palm size and/or pocket-size.

In the description below, it is described exemplarily how a motor assembly may actuate a pre-determined distance of movement of a syringe plunger which in turn correlates to a pre-determined volume for the syringe based on the displacement or linear distance.

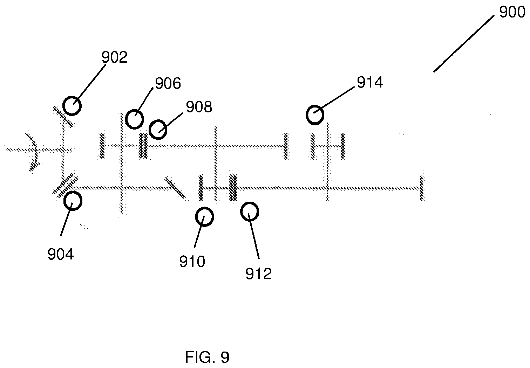

FIG. 9 is a schematic diagram for illustrating a gear train assembly 900. The first motor assembly 406 of FIG. 4B and the second motor assembly 512 of FIG. 5E utilize gear train assemblies substantially identical to the gear train assembly 900. In the gear train assembly 900, each of the gears 902, 904, 906, 908, 910, 912, 914 may be, but is not limited to, a nylon gear. The gear module of each of the gears 902, 904, 906, 908, 910, 912, 914 is 0.5. The gear ratio of the motor to the drive shaft is 297.92:1. For every revolution made by the motor, 3 counts are recorded.

The number of teeth on each of the gears 902, 904, 906, 908, 910, 912, 914 are denoted by $N_{902}$, $N_{904}$, $N_{906}$, $N_{908}$, $N_{910}$, $N_{912}$ and $N_{914}$ respectively as follows: Number of Teeth: $N_{902}$=20; $N_{904}$=22; $N_{906}$=10; $N_{908}$=28; $N_{910}$=8; $N_{912}$=38; $N_{914}$=8

$$\frac{\omega_{902}}{\omega_{914}} = \left(-\frac{22}{20}\right)\left(-\frac{28}{10}\right)\left(-\frac{38}{8}\right) = -14.63$$

Therefore, the mechanical advantage from gear 902 to gear 914 is 14.63.

For gear 914:

$$diameter, d = N \times module = 8 \times 0.5 = 4mm$$

$$1 \, rev = \pi \times d = 12.566mm \, (Linear \, displacement)$$

For a syringe, in order to allow about 5ml of body fluid to flow into the syringe, the plunger member may be linearly displaced by about 40mm. For a linear displacement of 40mm, gear 914 therefore makes:

$$40 \div 12.566mm = 3.1832 \, revolutions$$

It is calculated that 3.1832 revolutions on gear 914 yields:

$$3.1832 \times 14.63 \times 297.92 \times 3 = 41622.59625 \, counts \, on \, the \, second \, motor \, assembly$$

The correlationship between the second motor assembly and linear displacement of the syringe is as follows:

$$\frac{41622.59625}{40} = 1040.5649 \approx \frac{1040.6 \, counts}{mm}$$

Therefore, with the above correlation, it may be provided to allow a pre-determined volume of fluid into a syringe based on linear distance moved of the plunger of the syringe caused by the corresponding motor.

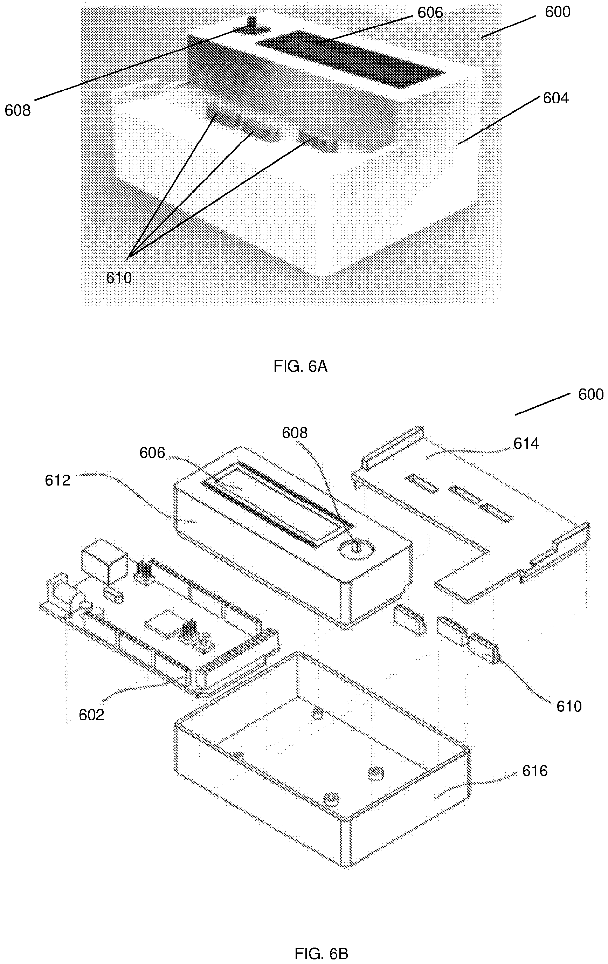

FIG. 6A is a perspective view of a docking module of a body fluid drainage device in an exemplary embodiment. FIG. 6B is an exploded view of the docking module. In the exemplary embodiment, the docking module 600 functions substantially identically to the docking module 220 described with reference to Fig. 2. In the exemplary embodiment, the docking module 600 comprises a processing module 602, a third housing 604, a display component 606, an input component 608 and pin connectors e.g. 610. The processing module 602 comprises a microcontroller. The third housing 604 comprises a top cover 612, a middle cover 614 and a bottom cover 616. The display component 606 and the input component 608 are disposed on the top cover 612. The display component 606 may be, but is not limited to, a liquid crystal display (LCD) to visually display parameters or settings inputted by a user/giver and/or the current operating status of a body fluid drainage device. The display component 606 may also display fluid volume drained in real-time. The input component 608 may be, but is not limited to, a joystick for a user/caregiver to input to the processing module 602 a desired body fluid drainage rate and/or a pre-determined total volume of fluid to be drained from a body cavity. For example, one movement of the joystick indicates a desired body fluid drainage rate increment of 5 milliliters per second. The middle cover 614 is configured with openings to receive three pin connectors e.g. 610. A channel switching module and a fluid drainage module may be removably coupled to the processing module 602 via the pin connectors e.g. 610. For example, two pin connectors are used to couple a fluid drainage module to the processing module 602 and one pin connector is used to couple a channel switching module to the processing module 602.

In addition, a power source (not shown) is also housed in the docking module 600. The power source may be, but is not limited to, one or more lithium-ion polymer batteries. The power source may also power the channel switching module and the fluid drainage module. Furthermore, the docking module 600 may also comprise a termination actuator (not shown), such as a button, to allow a user to manually terminate/stop fluid drainage.

In the exemplary embodiment, the length, breadth and height of an exterior of the third housing 604 of the docking module 600 are about 110 mm, 80 mm and 47 mm respectively.

Therefore, the docking module 600 is suitably portable and smaller than a typical palm size and/or pocket-size when a channel switching module (such as the channel switching module 400) and a fluid drainage module (such as the fluid drainage module 500) are coupled to the docking module 600.

In an example implementation, the processing module comprises a motor controller. The processing module is capable of using voltage and/or the pulse-width modulation variations to control a motor housed in a fluid drainage module. For example, the motor controller is capable of converting a signal from a processor to a Pulse-width Modulated (PWM) voltage to cause the motor (e.g. motor assembly 512 of FIG. 5E) in the fluid drainage module to rotate at a pre-determined speed/rate and direction determined by the processing module. The motor is coupled to a series of gears (e.g. gear rack 514 of FIG. 5E) which causes movement of the force transmittal member.

In medical literature, the maximum/total volume of fluid drainage from the pleural cavity that is acceptable medically is 1.5 liters/day. To prevent occurrences of complications such as RPE, the current British Thoracic Society guidelines suggest that less than 1.5 liters of pleural fluid are to be drained in a day. In practical settings, the drainage of 1.5 liter of pleural fluid from the pleural cavity is carried out in one hour. Fluid drainage rate that is higher than 1.5 liters per hour is thus considered too fast medically. Furthermore, the normal pleural pressure in a mammalian body is about -5cmH$_2$O. The negative limit of pleural pressure wherein medical practitioners widely believe that risks and complications may more likely occur for a mammalian body is -20cmH$_2$O. Certain medical studies also show that the risk of encountering re-expansion pulmonary edema (RPE) may be reduced by maintaining a pleural pressure of higher than -20cmH$_2$O. In another exemplary embodiment, a body fluid drainage device may be provided such that not only is the flow rate controllable, the body fluid drainage device may also stop draining body fluid from a body cavity upon detection of a pre-determined threshold cavity pressure.

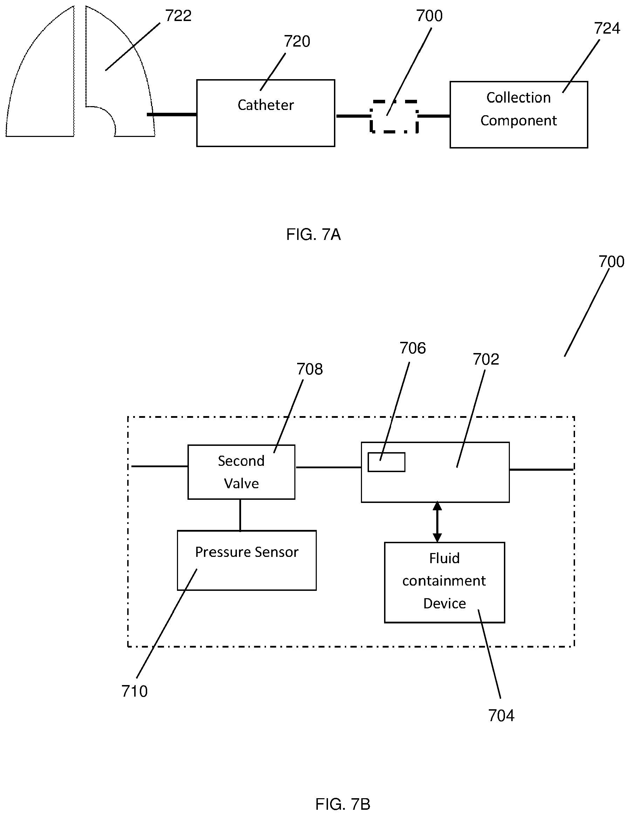

FIG. 7A is a schematic drawing illustrating a body fluid drainage device in use in an exemplary embodiment. FIG. 7B is a schematic drawing of the body fluid drainage device. In the exemplary embodiment, the body fluid drainage device 700 comprises a first channel switching module 702 that is coupled to a fluid containment device 704. The fluid containment device 704 may be separately provided to the body fluid drainage device 700. The fluid containment device 704 may be, for example, a syringe. The first channel switching module 702 comprises a first valve 706. The body fluid drainage device 700 further comprises a second valve 708 which is coupled along the same channel as the first valve 706. The body fluid drainage device 700 is removably coupled via a catheter 720, for example a chest drain tube, to a body cavity 722 such as a pleural cavity. The body fluid drainage device 700 is further removably coupled to a collection component 724, for example a chest drain box or bag.

In the exemplary embodiment, a barometer or pressure sensor 710 is coupled to the second valve 708 to measure the pressure of the body cavity 722 through a fluid flow along the channel towards the first valve 706.

In another exemplary embodiment, in addition or as an alternative, force sensors may be removably attached to a plunger member of a syringe to measure the force exerted by body fluid on the plunger member and therefore, the pressure of the body cavity is measured. For example, the body fluid within the syringe exerts a force on the plunger member if there is a pressure difference between the pressure in the body cavity and atmospheric pressure. Force sensors may be attached on two ends of the plunger member (for example, at one end of the plunger member which is located internally within the fluid containment device and at another end of the plunger member which is located external of the fluid containment device) to take into account both the translational (pushing or pulling) actions of the syringe (i.e. the body fluid being discharged from the syringe into the collection component and the body fluid flowing from the body cavity into the syringe).

When the force exerted on the plunger is determined, the pressure of the body cavity is calculated by using the following formula:

$$Pressure = \frac{Force}{Surface\ area\ of\ plunger\ that\ is\ in\ contact\ with\ the\ fluid}$$

In the exemplary embodiments utilizing a pressure or force sensor, the body fluid drainage device may additionally be configured to stop the flow rate actuator and to stop the fluid drainage based on a pre-determined threshold pressure. For example, when a catheter is obstructed by residues or blockages, the pressure measured may be significantly high and exceeds the pre-determined threshold pressure. The pressure or force sensor may signal the processing module to prevent the motor assembly from being overloaded. The force sensor thus acts as a safety feature to stop the flow rate actuator from draining the body fluid from the body cavity.

For another example, when the pressure sensor 710 of FIG. 7B detects that the pleural pressure reaches -20 cmH$_2$O that is designated as a threshold pressure of the body cavity, the body fluid drainage device 700 is instructed by the processing module to cease or stop drainage of body fluid from the pleural cavity.

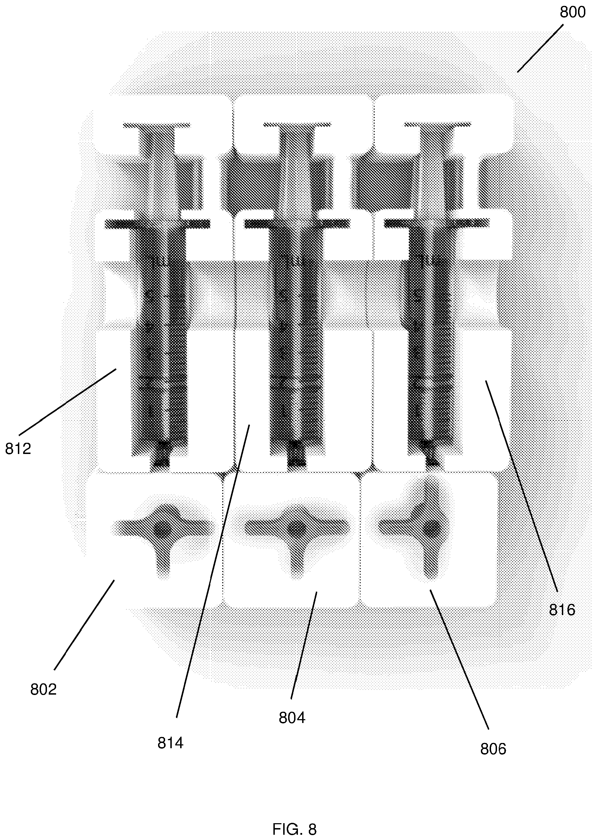

FIG. 8 is a top view of a body fluid drainage system for draining a fluid from a body cavity in an exemplary embodiment. In the exemplary embodiment, the body fluid drainage system 800 comprises a plurality of channel switching modules 802, 804, 806 and a plurality of fluid drainage modules 812, 814, 816. The fluid drainage modules 812, 814, 816 are respectively coupled to the channel switching modules 802, 804, 806. The plurality of channel switching modules 802, 804, 806 and the plurality of fluid drainage modules 812, 814, 816 are respectively coupled to a processing module (not shown). The body fluid drainage system may be customized according to the requirements of a user/caregiver. For example, each of the channel switching modules 802, 804, 806 may function separately from each other upon instruction by the processing module. Each of the fluid drainage modules 812, 814, 816 may function separately from each other upon instruction by the processing module.

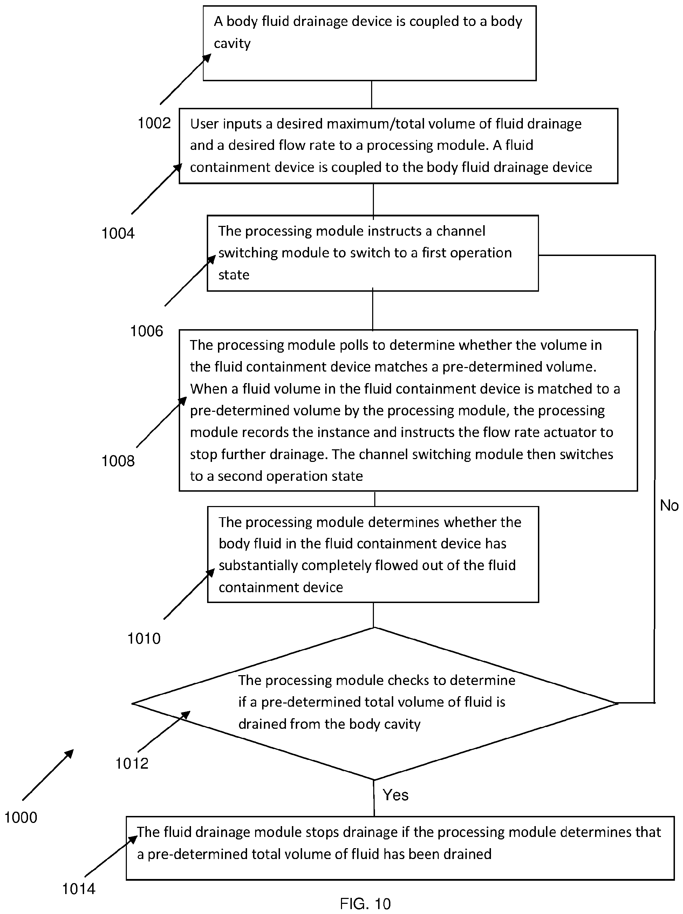

FIG. 10 is a schematic flowchart 1000 for illustrating an operation of a body fluid drainage device in an exemplary embodiment.

At step 1002, a body fluid drainage device is coupled to a body cavity using e.g. a catheter.

At step 1004, a user inputs a desired maximum/total volume of fluid drainage and a desired flow rate (e.g. in milliliters per second or ml/s) to a processing module. The flow rate and total volume may be prescriptions from a medical practitioner. A fluid containment device is coupled to the body fluid drainage device.

At step 1006, the processing module instructs a channel switching module to switch to a first operation state. In the first operation state, the channel switching module is used to control fluid flow between a body cavity and the fluid containment device. Body fluid is allowed to flow from the body cavity through a first channel into the fluid containment device. The processing module controls the flow rate of the fluid into the fluid containment device using a flow rate actuator and based on the inputted pre-determined flow rate.

At step 1008, the processing module polls to determine whether the volume in the fluid containment device matches a pre-determined volume. When a fluid volume in the fluid containment device is matched to a pre-determined volume by the processing module, the processing module records the instance and instructs the flow rate actuator to stop further drainage. The processing module instructs the channel switching module to switch from the first operation state to a second operation state. In the second operation state, the processing module uses the channel switching module to allow fluid flow from the fluid containment device. Thus, body fluid is allowed to flow out of or be expelled from the fluid containment device through a second channel. The flow of fluid out of the fluid containment device is instructed by the processing module using the flow rate actuator in reverse motion.

At step 1010, the processing module determines whether the body fluid in the fluid containment device has substantially completely flowed out of the fluid containment device.

If the processing module determines that the body fluid in the fluid containment device has substantially completely flowed out of the fluid containment device, at step 1012, the processing module checks to determine if a pre-determined total volume of fluid is drained from the body cavity based on a number of times the pre-determined volume of fluid has been obtained in the fluid containment device.

For example, in a scenario where a total volume of 25ml of fluid is desired to be drained from a body cavity and the pre-determined volume of fluid in the fluid containment device is 5ml, the processing module is capable of determining that the total volume of 25ml of fluid has been drained if the processing module records that the pre-determined volume of fluid in the fluid containment device has been obtained 5 times.

If the result is affirmative at step 1012, i.e. the pre-determined total volume of fluid is drained from the body cavity, the processing module instructs the fluid drainage module at step 1014 to stop fluid drainage from the body cavity to the fluid containment device.

If the result is negative at step 1012, i.e. the pre-determined total volume of fluid drained from the body cavity is not yet achieved, the process loops back to step 1006. That is, the processing module instructs the channel switching module to switch to allow fluid flow between the body cavity and the fluid containment device to allow a next cycle of fluid drainage e.g. to switch between the first operation state and the second operation state.

In an alternative implementation, the check of the processing module at step 1012 may be performed instead after the step 1006 i.e. after a match to a pre-determined volume in the fluid containment device. Thus, in the alternative implementation, if it is determined that the pre-determined total volume of fluid is drained from the body cavity, fluid in the fluid containment device may remain in the fluid containment device, and the fluid drainage process may stop.

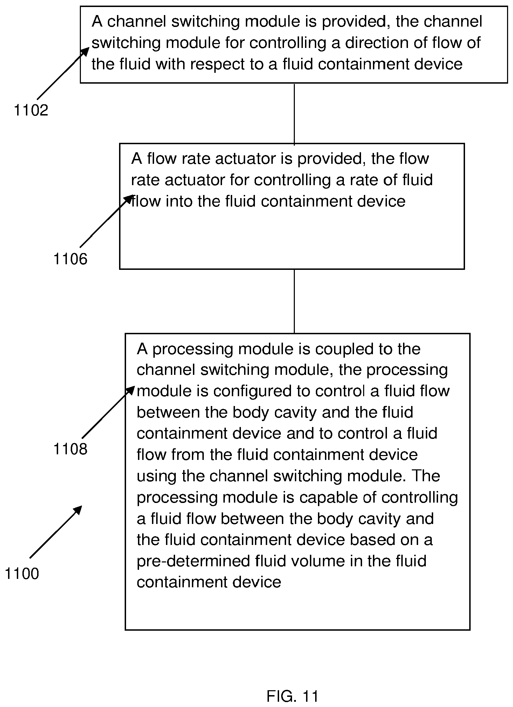

FIG. 11 is a schematic flow chart for illustrating a method of forming a body fluid drainage device for draining a fluid from a body cavity in an exemplary embodiment. At step 1102, a channel switching module is provided, the channel switching module for controlling a direction of flow of the fluid with respect to a fluid containment device. At step 1106, a flow rate actuator is provided, the flow rate actuator for controlling a rate of fluid flow into the fluid containment device. At step 1108, a processing module is coupled to the channel switching module, the processing module is configured to control a fluid flow between the body cavity and the fluid containment device and to control a fluid flow from the fluid containment device using the channel switching module. The processing module is capable of controlling a fluid flow between the body cavity and the fluid containment device based on a pre-determined fluid volume in the fluid containment device.

The fluid containment device may be separately provided and coupled to the channel switching module and the flow rate actuator.

The processing module may be further configured to determine whether a total volume of fluid has been drained from the body cavity. The determination is based on a number of times the pre-determined fluid volume in the fluid containment device has been obtained. The number of times the pre-determined fluid volume is obtained in the fluid containment device may correspond to the number of switches of the channel switching module to control fluid flow from the fluid containment device.

In the described exemplary embodiments, the body fluid drainage device may allow fluid flow/communication from a body cavity to be controlled at a pre-determined flow rate. The desired maximum/total volume of fluid drainage may also be monitored automatically without human intervention. Therefore, the body fluid drainage device may allow the fluid drainage process to be performed or carried out without constant monitoring by a caregiver. The body fluid drainage device may even be self-used e.g. by a patient, together with a prescription from a medical practitioner.

In the described exemplary embodiments, the body fluid drainage device may be portable. The size of the various components of the body fluid drainage device are substantially smaller than palm-size and/or pocket-size. Therefore, portability of the body fluid drainage device may be improved. For example, a user may carry the body fluid drainage device around in a clothing pocket and therefore, may no longer be confined to a bed or couch for fluid drainage.

Furthermore, the components of the body fluid drainage device, for example the fluid containment device, may be interchangeable. Therefore, costs may be effectively reduced since it may be decided that only sterile parts (and not the entire drainage device) is to be changed. In addition, the fluid containment device may be interchangeable for different volumes.

In the described exemplary embodiments, the components of the body fluid drainage device, for example the channel switching module, the fluid containment device and the processing module, may be individually coupled to each other. This may allow a caregiver to set up the body fluid drainage device according to a patient's individual requirements/conditions without incurring a high cost of modification.

It has also been recognized by the inventors that the body fluid drainage device of described exemplary embodiments may also be used as a data logger to collect pressure data points that may be used for research purposes.

In the described exemplary embodiments, it is appreciated that the channel switching module is capable of switching between at least two channels to allow fluid to flow into or out of a fluid containment device. Even though a channel switching module with a valve or a 3-way tap has been described in some exemplary embodiments, it will be appreciated that the exemplary embodiments are not limited as such. For example, the channel switching module may be capable of parallel switching between a channel coupled to a body cavity and a channel coupled to a storage component, to couple the fluid containment device to the respective channel.

In the described exemplary embodiments, the pre-determined volume of fluid in the fluid containment device is a known, expected or projected volume. The actual drained fluid may contain bubbles but is appreciated to be substantially close to the expected volume of drained fluid.

In some exemplary embodiments, the processing module may stop fluid drainage by instructing a flow rate actuator to stop operation. In addition or as an alternative, the processing module may instruct the channel switching module to switch out of two channels into a third channel that is not being used. For example, if the channel switching module uses a three way tap, the processing module may instruct the tap to be switched to a third channel that is not being used.

In some exemplary embodiments, the processing module is capable of changing the pre-determined volume for a fluid containment device such that the pre-determined total volume of fluid drainage is achieved. For example, in a scenario where a total volume of 23ml of fluid is desired to be drained from a body cavity and a fluid containment device has a maximum volume of 5ml, the processing module is capable of instructing the pre-determined volume for four cycles of fluid drainage to be 5ml (the maximum volume of the fluid containment device) and instructing the pre-determined volume for one cycle to be 3ml. Thus, over the five cycles of drainage, the desired total volume of 23ml of fluid is drained from a body cavity.

In some exemplary embodiments, a maximum volume of a fluid containment device may be the volume as indicated on the fluid containment device. However, it is appreciated that the exemplary embodiments are not limited as such. For example, if a fluid containment device is in the form of a syringe that indicates a calibrated scale up to 5ml, the maximum volume of the syringe may be determined to be about 10% more than the indicated scale. In some exemplary embodiments, the indications of a maximum volume on the fluid containment device may substantially be the actual maximum volume of the fluid containment device.

The terms "coupled" or "connected" as used in this description are intended to cover both directly connected or connected through one or more intermediate means, unless otherwise stated.

The description herein may be, in certain portions, explicitly or implicitly described as algorithms and/or functional operations that operate on data within a computer memory or an electronic circuit. These algorithmic descriptions and/or functional operations are usually used by those skilled in the information/data processing arts for efficient description. An algorithm is generally relating to a self-consistent sequence of steps leading to a desired result. The algorithmic steps can include physical manipulations of physical quantities, such as electrical, magnetic or optical signals capable of being stored, transmitted, transferred, combined, compared, and otherwise manipulated.

Further, unless specifically stated otherwise, and would ordinarily be apparent from the following, a person skilled in the art will appreciate that throughout the present specification, discussions utilizing terms such as "scanning", "calculating", "determining", "replacing", "generating", "initializing", "outputting", and the like, refer to action and processes of an instructing processor/computer system, or similar electronic circuit/device/component, that manipulates/processes and transforms data represented as physical quantities within the described system into other data similarly represented as physical quantities within the system or other information storage, transmission or display devices etc.

The description also discloses relevant device/apparatus for performing the steps of the described methods. Such apparatus may be specifically constructed for the purposes of the methods, or may comprise a general purpose computer/processor or other device selectively activated or reconfigured by a computer program stored in a storage member. The algorithms and displays described herein are not inherently related to any particular computer or other apparatus. It is understood that general purpose devices/machines may be used in accordance with the teachings herein. Alternatively, the construction of a specialized device/apparatus to perform the method steps may be desired.

In addition, it is submitted that the description also implicitly covers a computer program, in that it would be clear that the steps of the methods described herein may be put into effect by computer code. It will be appreciated that a large variety of programming languages and coding can be used to implement the teachings of the description herein. Moreover, the computer program if applicable is not limited to any particular control flow and can use different control flows without departing from the scope of the disclosure.

Furthermore, one or more of the steps of the computer program if applicable may be performed in parallel and/or sequentially. Such a computer program if applicable may be stored on any computer readable medium. The computer readable medium may include storage devices such as magnetic or optical disks, memory chips, or other storage devices suitable for interfacing with a suitable reader/general purpose computer. In such instances, the computer readable storage medium is non-transitory. Such storage medium also covers all computer-readable media e.g. medium that stores data only for short periods of time and/or only in the presence of power, such as register memory, processor cache and Random Access Memory (RAM) and the like. The computer readable medium may even include a wired medium such as exemplified in the Internet system, or wireless medium such as exemplified in bluetooth technology. The computer program when loaded and executed on a suitable reader effectively results in an apparatus that can implement the steps of the described methods.

The exemplary embodiments may also be implemented as hardware modules. A module is a functional hardware unit designed for use with other components or modules. For example, a module may be implemented using digital or discrete electronic components, or it can form a portion of an entire electronic circuit such as an Application Specific Integrated Circuit (ASIC). A person skilled in the art will understand that the exemplary embodiments can also be implemented as a combination of hardware and software modules.

Additionally, when describing some embodiments, the disclosure may have disclosed a method and/or process as a particular sequence of steps. However, unless otherwise required, it will be appreciated the method or process should not be limited to the particular sequence of steps disclosed. Other sequences of steps may be possible. The particular order of the steps disclosed herein should not be construed as undue limitations. Unless otherwise required, a method and/or process disclosed herein should not be limited to the steps being carried out in the order written. The sequence of steps may be varied and still remain within the scope of the disclosure.

Further, in the description herein, the word "substantially" whenever used is understood to include, but not restricted to, "entirely" or "completely" and the like. In addition, terms such as "comprising", "comprise", and the like whenever used, are intended to be non-restricting descriptive language in that they broadly include elements/components recited after such terms, in addition to other components not explicitly recited. Further, terms such as "about", "approximately" and the like whenever used, typically means a reasonable variation, for example a variation of +/- 5% of the disclosed value, or a variance of 4% of the disclosed value, or a variance of 3% of the disclosed value, a variance of 2% of the disclosed value or a variance of 1% of the disclosed value.

Furthermore, in the description herein, certain values may be disclosed in a range. The values showing the end points of a range are intended to illustrate a preferred range. Whenever a range has been described, it is intended that the range covers and teaches all possible sub-ranges as well as individual numerical values within that range. That is, the end points of a range should not be interpreted as inflexible limitations. For example, a description of a range of 1% to 5% is intended to have specifically disclosed sub-ranges 1% to 2%, 1% to 3%, 1% to 4%, 2% to 3% etc., as well as individually, values within that range such as 1%, 2%, 3%, 4% and 5%. The intention of the above specific disclosure is applicable to any depth/breadth of a range.

It will be appreciated by a person skilled in the art that other variations and/or modifications may be made to the specific embodiments without departing from the scope of the invention as defined by the appended claims. The present embodiments are, therefore, to be considered to be illustrative and not restrictive.

## Claims

1. A body fluid drainage device (100, 200, 300) for draining a fluid from a body cavity (112, 312), the device comprising,

   a channel switching module (102, 202, 302, 400) for coupling to a fluid containment device (104, 204, 304, 520), the channel switching module (102, 202, 302, 400) for controlling a direction of flow of the fluid with respect to the fluid containment device (104, 204, 304, 520);

a flow rate actuator (106) for coupling to the fluid containment device (104, 204, 304, 520), the flow rate actuator (106) for controlling a rate of fluid flow into the fluid containment device (104, 204, 304, 520);

a processing module (108, 602) coupled to the channel switching module (102, 202, 302, 400), the processing module (108, 602) being configured to control a fluid flow between the body cavity (112, 312) and the fluid containment device (104, 204, 304, 520) and to control a fluid flow from the fluid containment device (104, 204, 304, 520) using the channel switching module (102, 202, 302, 400);

wherein the processing module (108, 602) is capable of controlling the fluid flow between the body cavity (112, 312) and the fluid containment device (104, 204, 304, 520) based on a pre-determined fluid volume in the fluid containment device (104, 204, 304, 520); and

wherein the fluid containment device (104, 204, 304, 520) comprises a syringe (304, 520).

2. The body fluid drainage device (100, 200, 300) of claim 1, further comprising the fluid containment device (104, 204, 304, 520), the fluid containment device (104, 204, 304, 520) being removably coupled to the flow rate actuator (106).

3. The body fluid drainage device (100, 200, 300) of claim 2, wherein the fluid containment device (104, 204, 304, 520) is removably coupled to the channel switching module (102, 202, 302, 400).

4. The body fluid drainage device (100, 200, 300) of any one of the preceding claims, wherein the channel switching module (102, 202, 302, 400), the flow rate actuator (106) or both are adapted to be removably coupled to the processing module (108, 602).

5. The body fluid drainage device (100, 200, 300) of any one of the preceding claims, wherein the processing module (108, 602) is configured to determine whether a fluid volume in the fluid containment device (104, 204, 304, 520) matches the pre-determined fluid volume, and if the fluid volume in the fluid containment device (104, 204, 304, 520) matches the pre-determined fluid volume, the processing module (108, 602) is configured to stop fluid flow between the body cavity (112, 312) and the fluid containment device (104, 204, 304, 520).

6. The body fluid drainage device (100, 200, 300) of claim 5, wherein the processing module (108, 602) is configured to stop fluid flow between the body cavity (112, 312) and the fluid containment device (104, 204, 304, 520) using the flow rate actuator (106), or using channel switching module (102, 202, 302, 400) or both.

7. The body fluid drainage device (100, 200, 300) of claims 5 or 6, wherein the determination of whether a fluid volume in the fluid containment device (104, 204, 304, 520) matches the pre-determined fluid volume is based on a maximum volume of the fluid containment device (104, 204, 304, 520).

8. The body fluid drainage device (100, 200, 300) of claims 5 or 6, wherein the determination of whether a fluid volume in the fluid containment device (104, 204, 304, 520) matches the pre-determined fluid volume is based on the rate of fluid flow controlled by the flow rate actuator (106).

9. The body fluid drainage device (100, 200, 300) of claim 8, wherein the rate of fluid flow controlled by the flow rate actuator (106) is correlated to an advancement distance of the fluid flow in the fluid containment device (104, 204, 304, 520).

10. The body fluid drainage device (100, 200, 300) of any one of the preceding claims, wherein the channel switching module (102, 202, 302, 400) is capable of switching between at least two channels (332, 334);

further wherein in a first operation state, the processing module (108, 602) is configured to instruct the channel switching module (102, 202, 302, 400) to switch to a first channel (332) to allow fluid communication from the body cavity (112, 312) to the fluid containment device (104, 204, 304, 520) through the first channel (332); and in a second operation state, the processing module (108, 602) is configured to instruct the channel switching module (102, 202, 302, 400) to switch to a second channel (334) to allow fluid communication from the fluid containment device (104, 204, 304, 520) through the second channel (334).

11. The body fluid drainage device (100, 200, 300) of claim 10, wherein in the second operation state, the processing module (108, 602) is configured to actuate expelling of fluid from the fluid containment device (104, 204, 304, 520) with an actuation of the flow rate actuator (106) in reverse motion.

12. The body fluid drainage device (100, 200, 300) of any one of the preceding claims, wherein the processing module (108, 602) is configured to determine whether a pre-determined total volume of fluid drainage has been achieved, said determination based on determining a number of times the pre-determined fluid volume in the fluid containment device (104, 204, 304, 520) is obtained.

13. The body fluid drainage device (100, 200, 300) of claim 12, wherein the said determination is further based on a number of switches of the channel switching module (102, 202, 302, 400) to allow fluid flow from the fluid containment device (104, 204, 304, 520).

14. The body fluid drainage device (100, 200, 300) of claims 12 or 13, further comprising an input module, the input module being configured to allow input to the processing module (108, 602) of the pre-determined total volume of fluid drainage and input to the processing module (108, 602) of the rate of fluid flow into the fluid containment device (104, 204, 304, 520).

15. The body fluid drainage device (100, 200, 300) of any one of the preceding claims, further comprising a storage component (114, 310), the storage component being coupled to the channel switching module (102, 202, 302, 400) for receiving fluid flow from the fluid containment device (104, 204, 304, 520).

16. The body fluid drainage device (100, 200, 300) of any one of the preceding claims, further comprising a pressure sensor (710) coupled to the channel switching module (102, 202, 302, 400) for measuring a pressure in the body cavity (112, 312).

17. The body fluid drainage device (100, 200, 300) of any one of the preceding claims, further comprising one or more force sensors coupled to the fluid containment device (104, 204, 304, 520) for measuring a pressure in the body cavity (112, 312).

18. The body fluid drainage device (100, 200, 300) of claims 16 or 17, wherein the processing module (108, 602) is further configured to control the fluid flow between the body cavity (112, 312) and the fluid containment device (104, 204, 304, 520) based on the measured pressure.

19. The body fluid drainage device (100, 200, 300) of any one of the preceding claims, wherein the flow rate actuator (106) comprises a force transmittal member (506) coupled to the fluid containment device (104, 204, 304, 520) and coupled to the processing module (108, 602); wherein the processing module (108, 602) is configured to control the fluid flow into the fluid containment device (104, 204, 304, 520) and expelled from the fluid containment device (104, 204, 304, 520) via a displacement of the force transmittal member (506).


**Patentansprüche**

1. Körperflüssigkeitsdrainagevorrichtung (100, 200, 300) zum Ableiten einer Flüssigkeit aus einem Körperhohlraum (112, 312), wobei die Vorrichtung umfasst,

ein Kanalumschaltmodul (102, 202, 302, 400) zum Ankoppeln an eine Flüssigkeitsbehältervorrichtung (104, 204, 304, 520), wobei das Kanalumschaltmodul (102, 202, 302, 400) zum Steuern einer Strömungsrichtung der Flüssigkeit in Bezug auf die Flüssigkeitsbehältervorrichtung (104, 204, 304, 520) dient;
einen Durchflussmengenaktuator (106) zum Ankoppeln an die Flüssigkeitsbehältervorrichtung (104, 204, 304, 520), wobei der Durchflussmengenaktuator (106) zur Steuerung einer Durchflussmenge der Flüssigkeit in die Flüssigkeitsbehältervorrichtung (104, 204, 304, 520) dient;
ein Verarbeitungsmodul (108, 602), das an das Kanalumschaltmodul (102, 202, 302, 400) gekoppelt ist, wobei das Verarbeitungsmodul (108, 602) so konfiguriert ist, dass es einen Flüssigkeitsfluss zwischen der Körperhöhle (112, 312) und der Flüssigkeitsbehältervorrichtung (104, 204, 304, 520) steuert und einen Flüssigkeitsfluss aus der Flüssigkeitsbehältervorrichtung (104, 204, 304, 520) unter Verwendung des Kanalumschaltmoduls (102, 202, 302, 400) steuert;
wobei das Verarbeitungsmodul (108, 602) in der Lage ist, den Flüssigkeitsfluss zwischen der Körperhöhle (112, 312) und der Flüssigkeitsbehältervorrichtung (104, 204, 304, 520) basierend auf einem vorbestimmten Flüssigkeitsvolumen in der Flüssigkeitsbehältervorrichtung (104, 204, 304, 520) zu steuern; und
wobei die Flüssigkeitsbehältervorrichtung (104, 204, 304, 520) eine Spritze (304, 520) umfasst.

**2.** Körperflüssigkeitsdrainagevorrichtung (100, 200, 300) gemäß Anspruch 1, weiterhin umfassend die Flüssigkeitsbehältervorrichtung (104, 204, 304, 520), wobei die Flüssigkeitsbehältervorrichtung (104, 204, 304, 520) lösbar an den Durchflussmengenaktuator (106) gekoppelt ist.

**3.** Körperflüssigkeitsdrainagevorrichtung (100, 200, 300) gemäß Anspruch 2, wobei die Flüssigkeitsbehältervorrichtung (104, 204, 304, 520) lösbar an das Kanalumschaltmodul (102, 202, 302, 400) gekoppelt ist.

**4.** Körperflüssigkeitsdrainagevorrichtung (100, 200, 300) gemäß irgendeinem der vorhergehenden Ansprüche, wobei das Kanalumschaltmodul (102, 202, 302, 400), der Durchflussmengenaktuator (106) oder beide so ausgelegt sind, dass sie lösbar an das Verarbeitungsmodul (108, 602) gekoppelt werden können.

**5.** Körperflüssigkeitsdrainagevorrichtung (100, 200, 300) gemäß irgendeinem der vorhergehenden Ansprüche, wobei das Verarbeitungsmodul (108, 602) konfiguriert ist, um zu bestimmen, ob ein Flüssigkeitsvolumen in der Flüssigkeitsbehältervorrichtung (104, 204, 304, 520) mit dem vorbestimmten Flüssigkeitsvolumen übereinstimmt, und wenn das Flüssigkeitsvolumen in der Flüssigkeitsbehältervorrichtung (104, 204, 304, 520) mit dem vorbestimmten Flüssigkeitsvolumen übereinstimmt, ist das Verarbeitungsmodul (108, 602) konfiguriert, um den Flüssigkeitsfluss zwischen der Körperhöhle (112, 312) und der Flüssigkeitsbehältervorrichtung (104, 204, 304, 520) zu stoppen.

**6.** Körperflüssigkeitsdrainagevorrichtung (100, 200, 300) gemäß Anspruch 5, wobei das Verarbeitungsmodul (108, 602) so konfiguriert ist, dass es den Flüssigkeitsfluss zwischen der Körperhöhle (112, 312) und der Flüssigkeitsbehältervorrichtung (104, 204, 304, 520) unter Verwendung des Durchflussmengenaktuators (106) oder unter Verwendung des Kanalumschaltmoduls (102, 202, 302, 400) oder beidem stoppt.

**7.** Körperflüssigkeitsdrainagevorrichtung (100, 200, 300) gemäß Anspruch 5 oder 6, wobei die Bestimmung, ob ein Flüssigkeitsvolumen in der Flüssigkeitsbehältervorrichtung (104, 204, 304, 520) mit dem vorgegebenen Flüssigkeitsvolumen übereinstimmt, auf Grundlage eines maximalen Volumens der Flüssigkeitsbehältervorrichtung (104, 204, 304, 520) erfolgt.

**8.** Körperflüssigkeitsdrainagevorrichtung (100, 200, 300) gemäß Anspruch 5 oder 6, wobei die Bestimmung, ob ein Flüssigkeitsvolumen in der Flüssigkeitsbehältervorrichtung (104, 204, 304, 520) mit dem vorgegebenen Flüssigkeitsvolumen übereinstimmt, auf Grundlage der vom Durchflussmengenaktuator (106) gesteuerten Durchflussmenge der Flüssigkeit erfolgt.

**9.** Körperflüssigkeitsdrainagevorrichtung (100, 200, 300) gemäß Anspruch 8, wobei die vom Durchflussmengenaktuator (106) gesteuerte Durchflussmenge mit einer Voschubdistanz des Flüssigkeitsflusses in der Flüssigkeitsbehältervorrichtung (104, 204, 304, 520) korreliert ist.

**10.** Körperflüssigkeitsdrainagevorrichtung (100, 200, 300) gemäß irgendeinem der vorhergehenden Ansprüche, wobei das Kanalumschaltmodul (102, 202, 302, 400) in der Lage ist, zwischen mindestens zwei Kanälen (332, 334) umzuschalten;

wobei weiterhin in einem ersten Operationsstatus das Verarbeitungsmodul (108, 602) so konfiguriert ist, dass es das Kanalumschaltmodul (102, 202, 302, 400) instruiert, auf einen ersten Kanal (332) umzuschalten, um eine Flüssigkeitsübertragung von der Körperhöhle (112, 312) zu der Flüssigkeitsbehältervorrichtung (104, 204, 304, 520) durch den ersten Kanal (332) zu ermöglichen; und
in einem zweiten Operationsstatus das Verarbeitungsmodul (108, 602) so konfiguriert ist, dass es das Kanalumschaltmodul (102, 202, 302, 400) instruiert, auf einen zweiten Kanal (334) umzuschalten, um eine Flüssigkeitsübertragung von der Flüssigkeitsbehältervorrichtung (104, 204, 304, 520) durch den zweiten Kanal (334) zu ermöglichen.

**11.** Körperflüssigkeitsdrainagevorrichtung (100, 200, 300) gemäß Anspruch 10, wobei im zweiten Operationsstatus das Verarbeitungsmodul (108, 602) so konfiguriert ist, dass es das Ausstoßen von Flüssigkeit aus der Flüssigkeitsbehältervorrichtung (104, 204, 304, 520) mit einer Betätigung des Durchflussmengenaktuators (106) in umgekehrter Richtung aktiviert.

**12.** Körperflüssigkeitsdrainagevorrichtung (100, 200, 300) gemäß irgendeinem der vorhergehenden Ansprüche, wobei das Verarbeitungsmodul (108, 602) konfiguriert ist, um zu bestimmen, ob ein vorbestimmtes Gesamtvolumen der Flüssigkeitsdrainage erreicht wurde, wobei die Bestimmung auf der Ermittlung einer Anzahl von Malen des Erreichens des vorbestimmten Flüssigkeitsvolumens in der Flüssigkeitsbehältervorrichtung (104, 204, 304, 520) basiert.

13. Körperflüssigkeitsdrainagevorrichtung (100, 200, 300) gemäß Anspruch 12, wobei die genannte Bestimmung ferner auf einer Anzahl von Schaltungen des Kanalumschaltmoduls (102, 202, 302, 400) basiert, um einen Flüssigkeitsfluss aus der Flüssigkeitsbehältervorrichtung (104, 204, 304, 520) zu ermöglichen.

14. Körperflüssigkeitsdrainagevorrichtung (100, 200, 300) gemäß Anspruch 12 oder 13, die weiterhin ein Eingabemodul umfasst, wobei das Eingabemodul so konfiguriert ist, dass es die Eingabe des vorbestimmten Gesamtvolumens der Flüssigkeitsdrainage in das Verarbeitungsmodul (108, 602) und die Eingabe der Rate des Flüssigkeitsflusses in die Flüssigkeitsbehältervorrichtung (104, 204, 304, 520) in das Verarbeitungsmodul (108, 602) ermöglicht.

15. Körperflüssigkeitsdrainagevorrichtung (100, 200, 300) nach irgendeinem der vorhergehenden Ansprüche, weiterhin umfassend eine Speichervorrichtung (114, 310), wobei die Speichervorrichtung (114, 310) an das Kanalumschaltmodul (102, 202, 302, 400) gekoppelt ist, um einen Flüssigkeitsfluss aus der Flüssigkeitsbehältervorrichtung (104, 204, 304, 520) aufzunehmen.

16. Körperflüssigkeitsdrainagevorrichtung (100, 200, 300) nach irgendeinem der vorhergehenden Ansprüche, weiterhin umfassend einen mit dem Kanalumschaltmodul (102, 202, 302, 400) gekoppelten Drucksensor (710) zur Messung eines Drucks in der Körperhöhle (112, 312).

17. Körperflüssigkeitsdrainagevorrichtung (100, 200, 300) gemäß irgendeinem der vorhergehenden Ansprüche, weiterhin umfassend einen oder mehrere Kraftsensoren, die an die Flüssigkeitsbehältervorrichtung (104, 204, 304, 520) gekoppelt sind, um einen Druck in der Körperhöhle (112, 312) zu messen.

18. Körperflüssigkeitsdrainagevorrichtung (100, 200, 300) gemäß Anspruch 16 oder 17, wobei das Verarbeitungsmodul (108, 602) weiterhin so konfiguriert ist, dass es den Flüssigkeitsfluss zwischen der Körperhöhle (112, 312) und der Flüssigkeitsbehältervorrichtung (104, 204, 304, 520) basierend auf dem gemessenen Druck steuert.

19. Körperflüssigkeitsdrainagevorrichtung (100, 200, 300) gemäß irgendeinem der vorhergehenden Ansprüche, wobei der Durchflussmengenaktuator (106) ein Kraftübertragungselement (506) umfasst, das an die Flüssigkeitsbehältervorrichtung (104, 204, 304, 520) und an das Verarbeitungsmodul (108, 602) gekoppelt ist; wobei das Verarbeitungsmodul (108, 602) so konfiguriert ist, dass es den Flüssigkeitsfluss in die Flüssigkeitsbehältervorrichtung (104, 204, 304, 520) hinein und aus der Flüssigkeitsbehältervorrichtung (104, 204, 304, 520) heraus über eine Verschiebung des Kraftübertragungselements (506) steuert.

## Revendications

1. Dispositif de drainage de fluide corporel (100, 200, 300) pour drainer un fluide depuis une cavité corporelle (112, 312), le dispositif comprenant,

   un module de commutation de canal (102, 202, 302, 400) pour le couplage à un dispositif de retenue de fluide (104, 204, 304, 520), le module de commutation de canal (102, 202, 302, 400) pour contrôler une direction d'écoulement du fluide par rapport au dispositif de retenue de fluide (104, 204, 304, 520) ;
   un actionneur de débit (106) pour le couplage au dispositif de retenue de fluide (104, 204, 304, 520), l'actionneur de débit (106) pour contrôler un débit d'écoulement de fluide dans le dispositif de retenue de fluide (104, 204, 304, 520) ;
   un module de traitement (108, 602) couplé au module de commutation de canal (102, 202, 302, 400), le module de traitement (108, 602) étant configuré pour contrôler un écoulement de fluide entre la cavité corporelle (112, 312) et le dispositif de retenue de fluide (104, 204, 304, 520) et à contrôler écoulement d'un fluide depuis le dispositif de retenue de fluide (104, 204, 304, 520) en utilisant le module de commutation de canal (102, 202, 302, 400) ;
   dans lequel le module de traitement (108, 602) est capable de contrôler l'écoulement du fluide entre la cavité corporelle (112, 312) et le dispositif de retenue de fluide (104, 204, 304, 520) basé sur un volume de fluide prédéterminé dans le dispositif de retenue de fluide (104, 204, 304, 520) ; et
   dans lequel le dispositif de retenue de fluide (104, 204, 304, 520) comprend une seringue (304, 520).

2. Dispositif de drainage de fluide corporel (100, 200, 300) selon la revendication 1, comprenant en outre le dispositif de retenue de fluide (104, 204, 304, 520), le dispositif de retenue de fluide (104, 204, 304, 520) étant raccordé de manière amovible à l'actionneur de débit (106).

3. Dispositif de drainage de fluide corporel (100, 200, 300) selon la revendication 2, dans lequel le dispositif de retenue de fluide (104, 204, 304, 520) est raccordé de manière amovible au module de commutation de canal (102, 202, 302, 400).

4. Dispositif de drainage de fluide corporel (100, 200, 300) selon l'une des revendications précédentes, dans lequel le module de commutation de canal (102, 202, 302, 400), l'actionneur de débit (106) ou les deux à la fois sont adaptés pour être raccordés de manière amovible au module de traitement (108, 602).

5. Dispositif de drainage de fluide corporel (100, 200, 300) selon l'une des revendications précédentes, dans lequel le module de traitement (108, 602) est configuré pour déterminer si un volume de fluide dans le dispositif de retenue de fluide (104, 204, 304, 520) correspond au volume de fluide prédéterminé, et lorsque le volume de fluide dans le dispositif de retenue de fluide (104, 204, 304, 520) correspond au volume de fluide prédéterminé, le module de traitement (108, 602) est configuré pour arrêter l'écoulement de fluide entre la cavité corporelle (112, 312) et le dispositif de retenue de fluide (104, 204, 304, 520).

6. Dispositif de drainage de fluide corporel (100, 200, 300) selon la revendication 5, dans lequel le module de traitement (108, 602) est configuré pour arrêter l'écoulement de fluide entre la cavité corporelle (112, 312) et le dispositif de retenue de fluide (104, 204, 304, 520) en utilisant l'actionneur de débit (106) ou en utilisant le module de commutation de canal (102, 202, 302, 400) ou les deux.

7. Dispositif de drainage de fluide corporel (100, 200, 300) selon les revendications 5 ou 6, dans lequel la détermination de savoir si un volume de fluide dans le dispositif de retenue de fluide (104, 204, 304, 520) correspond au volume de fluide prédéterminé est basée sur un volume maximum du dispositif de retenue de fluide (104, 204, 304, 520).

8. Dispositif de drainage de fluide corporel (100, 200, 300) selon les revendications 5 ou 6, dans lequel le détermination de savoir si un volume de fluide dans le dispositif de retenue de fluide (104, 204, 304, 520) correspond au volume de fluide prédéterminé est basé sur le débit de fluide contrôlé par l'actionneur de débit (106).

9. Dispositif de drainage de fluide corporel (100, 200, 300) selon la revendication 8, dans lequel le débit de fluide contrôlé par l'actionneur de débit (106) est corrélé à une distance d'avancement de l'écoulement de fluide dans le dispositif de retenue de fluide (104, 204, 304, 520).

10. Dispositif de drainage de fluide corporel (100, 200, 300) selon l'une des revendications précédentes, dans lequel le module de commutation de canal (102, 202, 302, 400) est capable de basculer entre au moins deux canaux (332, 334) ;

dans lequel en outre dans un premier état de fonctionnement, le module de traitement (108, 602) est configuré pour ordonner au module de commutation de canal (102, 202, 302, 400) de basculer vers un premier canal (332) pour permettre une communication fluidique depuis la cavité corporelle (112, 312) vers le dispositif de retenue de fluide (104, 204, 304, 520) à travers le premier canal (332) ; et
dans un deuxième état de fonctionnement, le module de traitement (108, 602) est configuré pour ordonner au module de commutation de canal (102, 202, 302, 400) de basculer vers un deuxième canal (334) pour permettre la communication fluidique depuis le dispositif de retenue de fluide (104, 204, 304, 520) à travers le deuxième canal (334).

11. Dispositif de drainage de fluide corporel (100, 200, 300) selon la revendication 10, dans lequel, dans le deuxième état de fonctionnement, le module de traitement (108, 602) est configuré pour actionner l'expulsion du fluide du dispositif de retenue de fluide (104, 204, 304, 520) avec un actionnement de l'actionneur de débit (106) en mouvement inverse.

12. Dispositif de drainage de fluide corporel (100, 200, 300) selon l'une des revendications précédentes, dans lequel le module de traitement (108, 602) est configuré pour déterminer si le drainage d'un volume total prédéterminé de fluide a été effectué, ladite détermination étant basée sur une détermination d'un nombre de fois où le volume de fluide prédéterminé dans le dispositif de retenue de fluide (104, 204, 304, 520) est obtenu.

**13.** Dispositif de drainage de fluide corporel (100, 200, 300) selon la revendication 12, dans lequel ladite détermination est en outre basée sur un nombre de commutateurs du module de commutation de canal (102, 202, 302, 400) pour permettre un écoulement de fluide depuis le dispositif de retenue de fluide (104, 204, 304, 520).

**14.** Dispositif de drainage de fluide corporel (100, 200, 300) selon les revendications 12 ou 13, comprenant en outre un module d'entrée, le module d'entrée étant configuré pour permettre l'entrée au module de traitement (108, 602) du volume total prédéterminé de drainage de fluide et l'entrée dans le module de traitement (108, 602) du débit d'écoulement de fluide dans le dispositif de retenue de fluide (104, 204, 304, 520).

**15.** Dispositif de drainage de fluide corporel (100, 200, 300) selon l'une des revendications précédentes, comprenant en outre un composant de stockage (114, 310), l'élément de stockage étant couplé au module de commutation de canal (102, 202, 302, 400) pour recevoir l'écoulement de fluide du dispositif de retenue de fluide (104, 204, 304, 520).

**16.** Dispositif de drainage de fluide corporel (100, 200, 300) selon l'une des revendications précédentes, comprenant en outre un capteur de pression (710) couplé au module de commutation de canal (102, 202, 302, 400) pour mesurer une pression dans la cavité corporelle (112, 312).

**17.** Dispositif de drainage de fluide corporel (100, 200, 300) selon l'une des revendications précédentes, comprenant en outre un ou plusieurs capteurs de force couplé au dispositif de retenue de fluide (104, 204, 304, 520) pour mesurer une pression dans la cavité corporelle (112, 312).

**18.** Dispositif de drainage de fluide corporel (100, 200, 300) selon les revendications 16 ou 17, dans lequel le module de traitement (108, 602) est en outre configuré pour contrôler l'écoulement de fluide entre la cavité corporelle (112, 312) et le dispositif de retenue de fluide (104, 204, 304, 520) sur la base de la pression mesurée.

**19.** Dispositif de drainage de fluide corporel (100, 200, 300) selon l'une des revendications précédentes, dans lequel l'actionneur de débit (106) comprend un élément de transmission de force (506) raccordé au dispositif de retenue de fluide (104, 204, 304, 520) et couplé au module de traitement (108, 602) ; dans lequel le module de traitement (108, 602) est configuré pour s'opposer à l'écoulement de fluide dans le dispositif de retenue de fluide (104, 204, 304, 520) et pour expulser le fluide du dispositif de retenue de fluide (104, 204, 304, 520) par un déplacement de l'élément de transmission de force (506).

100

120

110

Body
cavity
112

Storage
component
114

120

Channel
switching
module
102

Fluid containment device
104

Flow rate actuator 106

Processing module
108

FIG. 1

FIG. 2

312

302

304

332

300

334

310

FIG. 3A

312

340

302

304

332

334

310

FIG. 3B

312

302

304

332

334

340

310

FIG. 3C

312

302

304

340

332

334

310

FIG. 3D

400

420

404

FIG. 4A

FIG. 4B

502

500

508

504

510

506

FIG. 5A

500

520

522

FIG. 5B

502
508
500
522
504
510
506

FIG. 5C

500
502
520
522
504
506

FIG. 5D

FIG. 5E

FIG. 5F

FIG. 5G

FIG. 6A

FIG. 6B

722

720

700

724

Catheter

Collection
Component

FIG. 7A

700

706

702

708

Second
Valve

Pressure Sensor

Fluid
containment
Device

710

704

FIG. 7B

FIG. 8

FIG. 9

A body fluid drainage device is coupled to a body cavity

1002 | User inputs a desired maximum/total volume of fluid drainage and a desired flow rate to a processing module. A fluid containment device is coupled to the body fluid drainage device

1004

The processing module instructs a channel switching module to switch to a first operation state

1006

The processing module polls to determine whether the volume in the fluid containment device matches a pre-determined volume. When a fluid volume in the fluid containment device is matched to a pre-determined volume by the processing module, the processing module records the instance and instructs the flow rate actuator to stop further drainage. The channel switching module then switches to a second operation state

1008

No

The processing module determines whether the body fluid in the fluid containment device has substantially completely flowed out of the fluid containment device

1010

The processing module checks to determine if a pre-determined total volume of fluid is drained from the body cavity

1012

1000

Yes

The fluid drainage module stops drainage if the processing module determines that a pre-determined total volume of fluid has been drained

1014

FIG. 10

A channel switching module is provided, the channel switching module for controlling a direction of flow of the fluid with respect to a fluid containment device

1102

A flow rate actuator is provided, the flow rate actuator for controlling a rate of fluid flow into the fluid containment device

1106

A processing module is coupled to the channel switching module, the processing module is configured to control a fluid flow between the body cavity and the fluid containment device and to control a fluid flow from the fluid containment device using the channel switching module. The processing module is capable of controlling a fluid flow between the body cavity and the fluid containment device based on a pre-determined fluid volume in the fluid containment device

1108

1100

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014194840 A1
- WO 2011037838 A1
- US 9186449 B2
- WO 2015197463 A1